# EUROPEAN PATENT APPLICATION

(11) **EP 2 460 877 A1**
(43) Date of publication of application: **06.06.2012**
(21) Application number: 11155453.1
(22) Date of filing: 22.02.2011
(51) Int. Cl.: C12N 5/0789, A61K 35/12, C12N 15/113

(54) **Modulation of CD133+ hematopoietic stem cells**

(30) Priority: 01.12.2010 EP 10193396
(71) Applicant: Miltenyi Biotec GmbH, 51429 Bergisch Gladbach (DE)
(72) Inventor: Bissels, Ute, 50823, Köln (DE); Bosio, Andreas, Dr., 50939, Köln (DE); Wild, Stefan, Dr., 50829, Köln (DE)
(74) Representative: Kisters, Michael Marcus

(57) **Abstract**

The invention refers to a method for modulating the differentiation of cells, comprising contacting said cells with one or more RNA sequences corresponding substantially to the RNA sequences of the miRNAs selected from the group consisting of miR-10a, miR-23a, miR-23b, miR-24, miR-29a, miR-29b, miR-29c, miR-99a, miR-125a-5p, miR-125b, miR-142-3p, miR-142-5p, miR-146a, miR-146b-5p, miR-191, miR-425, miR-484, miR-551b or the complement sequence thereof.

SEQUENCE LISTING

<110>  Miltenyi Biotec GmbH

<120>  Modulation of CD133+ hematopoietic stem cells

<130>  MIL_038EP2

<160>  18

<170>  PatentIn version 3.3

<210>  1
<211>  23
<212>  RNA
<213>  hsa-miR-10a MIMAT0000253

<400>  1
uacccuguag auccgaauuu gug                                              23

<210>  2
<211>  21
<212>  RNA
<213>  hsa-miR-23a MIMAT0000078

<400>  2
aucacauugc cagggauuuc c                                                21

<210>  3
<211>  21
<212>  RNA
<213>  hsa-miR-23b MIMAT0000418

<400>  3
aucacauugc cagggauuac c                                                21

<210>  4
<211>  22
<212>  RNA
<213>  hsa-miR-24 MIMAT0000080

<400>  4
uggcucaguu cagcaggaac ag                                               22

<210>  5
<211>  22
<212>  RNA
<213>  hsa-miR-29a MIMAT0000086

<400>  5
uagcaccauc ugaaaucggu ua                                               22

<210>  6
<211>  23
<212>  RNA
<213>  hsa-miR-29b MIMAT0000100

<400>  6
uagcaccauu ugaaaucagu guu                                              23

<210>  7
<211>  22
<212>  RNA

<213>  hsa-miR-29c MIMAT0000681

<400>  7
uagcaccauu ugaaaucggu ua                                                22


<210>  8
<211>  22
<212>  RNA
<213>  hsa-miR-99a MIMAT0000097

<400>  8
aacccguaga uccgaucuug ug                                                22


<210>  9
<211>  24
<212>  RNA
<213>  hsa-miR-125a-5p MIMAT0000443

<400>  9
ucccugagac ccuuuaaccu guga                                              24


<210>  10
<211>  22
<212>  RNA
<213>  hsa-miR-125b MIMAT0000423

<400>  10
ucccugagac ccuaacuugu ga                                                22


<210>  11
<211>  21
<212>  RNA
<213>  hsa-miR-142-5p MIMAT0000433

<400>  11
cauaaaguag aaagcacuac u                                                 21


<210>  12
<211>  23
<212>  RNA
<213>  hsa-miR-142-3p MIMAT0000434

<400>  12
uguaguguuu ccuacuuuau gga                                               23


<210>  13
<211>  22
<212>  RNA
<213>  hsa-miR-146a MIMAT0000449

<400>  13
ugagaacuga auuccauggg uu                                                22


<210>  14
<211>  22
<212>  RNA
<213>  hsa-miR-146b-5p MIMAT0002809

<400>  14
ugagaacuga auuccauagg cu                                                22

<210>  15
<211>  23
<212>  RNA
<213>  hsa-miR-191 MIMAT0000440

<400>  15
caacggaauc ccaaaagcag cug                                                23


<210>  16
<211>  23
<212>  RNA
<213>  hsa-miR-425 MIMAT0003393

<400>  16
aaugacacga ucacucccgu uga                                                23


<210>  17
<211>  22
<212>  RNA
<213>  hsa-miR-484 MIMAT0002174

<400>  17
ucaggcucag uccccucccg au                                                 22


<210>  18
<211>  21
<212>  RNA
<213>  hsa-miR-551b MIMAT0003233

<400>  18
gcgacccaua cuugguuuca g                                                  21

## Description

### Field of the invention

The present application relates to modulating the differentiation of hematopoietic stem and progenitor cells. More particularly, the application relates to the maintenance of self-renewal capability of CD133+ hematopoietic stem cells through microRNAs.

### Background of the invention

Blood contains a complex mixture of cells, such as erythrocytes, the oxygen-transporting cells, the white blood cells comprising the cells of immune response, such as lymphocytes (T cells, B cells, dendritic cells, etc.) and macrophages, as well as the platelets that trigger blood clotting in case of tissue damage. Hematopoietic stem cells (HSCs) generate all these cells and can thus be considered as being multipotent and capable of regenerating the complex hematopoietic system. HSCs give rise to more specialized progenitor cells (HPCs) with more limited differentiation potential, which are the progenitors of red blood cells, platelets, and the two main categories of white blood cells, the lymphoid and the myeloid progenitors.

CD133 - also known as prominin-1 (PROM1) - was originally found on HSCs and HPCs deriving from human fetal liver, bone marrow, peripheral blood and leukapheresis products from cytokine-mobilized donors (Weigmann et al. 1997; Yin et al. 1997). Further studies revealed that long-term culture-initiating cells (LTC-ICs), the most primitive human hematopoietic cells analyzable *in vitro,* are highly enriched for CD133⁺ cells (Matsumoto et al. 2000). Moreover, CD133⁺ cells in the quiescent phase of the cell cycle have a phenotype consistent with HSCs and have high repopulating activity (Boxall et al. 2009). As CD34⁺ cells can be generated *in vitro* from CD133⁺CD34⁻cells (Summers et al. 2004), CD133⁺ cells appear to be ancestral to CD34⁺cells. Gene expression profiles and repopulation assays provided further evidence for a more primitive phenotype of CD133⁺ cells compared to CD34⁺ cells (de Wynter et al. 1998; Hemmoranta et al. 2006). Most of the CD133⁺ cells (>99%) express CD34 and half of the CD34⁺ cells express CD133 (Matsumoto et al. 2000). Nonetheless, CD34⁺ CD38⁻ populations as well as the expression of CD133 both have been used to enrich for early hematopoietic progenitor cells (Buhring et al. 1999; Copland et al. 2006; Giebel et al. 2006).

Currently, CD133⁺ cells are used in stem cell-based therapy for treatment of leukaemia (Feller et al. 2005; Isidori et al. 2007), neurodegenerative diseases (Martinez et al. 2009), myocardial infarction (Stamm et al. 2003; Stamm et al. 2007) and liver regeneration (Furst et al. 2007).

HSCs, i.e. in particular CD133⁺ cells, have a tremendous potential to reconstitute the hematopoietic system and are therefore used in clinical transplantation protocols to treat a variety of malignant and non-malignant disorders. Existing limitations are the number of HSCs that can be isolated from sources such as bone marrow, mobilized peripheral blood and umbilical cord blood. If limited numbers of HSCs are used in transplantation settings, engraftment periods of 50 days or more are standard and lead to a high risk of serious infectious complications for the patients. Therefore, the ability to increase the number of HSCs in vitro would improve the rate of immune reconstitution and provide new treatment options. But growing HSCs in vitro has turned out to be surprisingly difficult.

MicroRNAs (miRNAs) are 21 to 23 nucleotides long non-coding RNAs that are negatively regulating target mRNAs on a post-transcriptional level. Each miRNA has the potential to bind hundreds of mRNAs and one mRNA can be targeted by multiple miRNAs (Bartel 2009), highlighting the importance of miRNAs in complex networks of gene expression regulation. It is estimated that >60% of the mammalian transcriptome is under miRNA control (Friedman et al. 2009). miRNAs have been shown to play a distinct role in many different cellular, developmental and physiological processes including hematopoiesis (Gangaraju and Lin 2009). The role of miRNAs in early hematopoiesis, mediated by subpopulations of hematopoietic stem (HSC) and progenitor (HPC) cells characterized by the expression of CD34 and/or CD133 surface markers, is not well understood.

Detailed analysis of miRNA expression in hematopoiesis showed that miRNAs fine tune essentially each step in hematopoiesis, as summarized in recent reviews (Gangaraju and Lin 2009; Xiao and Rajewsky 2009). However, most of the performed studies focused on the later steps of hematopoietic lineage differentiation whereas the early steps of HSC differentiation, e.g. the role of miRNAs in self-renewal of the LT-HSCs and ST-HSC as well as the function of miRNAs in multipotent progenitors, are currently mostly unknown.

Currently, the available miRNA profiles for human HSCs and HPCs are restricted to CD34⁺ cells from bone marrow, peripheral blood, mobilized peripheral blood and cord blood

(Georgantas et al. 2007; Merkerova et al. 2009) CD34⁺CD38⁻ cells from cord blood (Liao et al. 2008; Han et al. 2010).and CD133⁺ cells from mobilized peripheral blood (Jin et al. 2008).

Human HSCs and HPCs have a therapeutic use in transplantations, but the amount and purity of the cells obtainable with the known methods is limited. Either the total number of cells is too small and can not be enlarged sufficiently by proliferation or the cells differentiate to other unwanted cell types.

Object of the invention was to improve the access to these cells. Surprisingly, it was found that specific miRNAs can be used to modulate CD133⁺ hematopoietic stem cells by preventing the differentiation, allowing direct differentiation or dedifferentiation.

### Summary of the Invention

The microRNAs miR-10a, miR-23a, miR-23b, miR-24, miR-29a, miR-29b, miR-29c, miR-99a, miR-125a-5p, miR-125b, miR-142-3p, miR-142-5p, miR-146a, miR-146b-5p, miR-191, miR-425, miR-484 and miR-551b (Table 1) were significantly differentially expressed between CD133⁺ and CD34⁺CD133⁻ cells. Out of these 18 miRNAs 13 miRNAs (group 1 and 2) were significantly higher expressed in CD133⁺ compared to CD34⁺CD133⁻ cells. These 13 miRNAs could be divided into one group of miRNAs that were not expressed in CD34⁻CD133⁻ cells (group1: miR-10a, miR-99a, miR-125a-5p, miR125b, miR146a, miR-146b-5p, miR-551b) and another group where the expression in CD34⁻CD133⁻ cells was even higher than in CD133⁺ cells (group 2: miR-23a, miR-23b, miR-24, miR-29a, miR-29b, miR-29c). Five miRNAs (group 3: miR-191, miR-142-3p, miR142-5p, miR-425, miR-484) were significantly lower expressed in CD133⁺ cells as in CD34⁺CD133⁻ cells.

Object of the invention is a method for modulating the differentiation of cells, comprising contacting said cells with one or more RNA sequences corresponding substantially to the RNA sequences of the miRNAs selected from the group consisting of miR-10a, miR-23a, miR-23b, miR-24, miR-29a, miR-29b, miR-29c, miR-99a, miR-125a-5p, miR-125b, miR-142-3p, miR-142-5p, miR-146a, miR-146b-5p, miR-191, miR-425, miR-484, miR-551b or the complement sequence thereof.

A further object of the invention is a cell culture medium comprising one or more RNA sequences corresponding substantially to the RNA sequences of the miRNAs selected from the group consisting of miR-10a, miR-23a, miR-23b, miR-24, miR-29a, miR-29b, miR-29c, miR-99a, miR-125a-5p, miR-125b, miR-142-3p, miR-142-5p, miR-146a, miR-146b-5p, miR-191, miR-425, miR-484, miR-551b or the complement sequence thereof.

A further object of the invention is the use of one or more RNA sequences corresponding substantially to the RNA sequences of the miRNAs selected from the group consisting of miR-10a, miR-23a, miR-23b, miR-24, miR-29a, miR-29b, miR-29c, miR-99a, miR-125a-5p, miR-125b, miR-142-3p, miR-142-5p, miR-146a, miR-146b-5p, miR-191, miR-425, miR-484, miR-551b or the complement sequence thereof for the modulation of cells.

A further object of the invention is a cell composition obtainable by the method of the invention.

A further object of the invention is the use of the cell composition obtainable by the method of the invention for stem cell therapy.

Unless defined otherwise, technical and scientific terms used herein have the same meaning as commonly understood by one of ordinary skill in the art to which this invention belongs.

The terms "microRNAs" and "miRNA" as used herein refer to microRNA. The microRNAs, short non-coding RNAs of ~21 to 23 nucleotides in length, regulate target mRNAs post-transcriptionally. Up to now about 900 different miRNAs have been identified in the human genome (miRBase 15.0). Each miRNA has the potential to target hundreds of mRNAs and one mRNA can be targeted by multiple miRNAs (Lim et al. 2005), highlighting the importance of miRNAs in complex networks of gene expression regulation. Other main characteristics of miRNAs are their evolutionary conservation and unique biogenesis as they are specifically processed from precursors with a hairpin structure (Ambros et al. 2003).

The processes of miRNA biogenesis and target mRNA repression have been studied intensively during the last years (Winter et al. 2009). miRNA genes are transcribed by either RNA polymerase II or RNA polymerase III into primary miRNA transcripts (pri-miRNA). The pri-miRNA is next endonucleolytically cleaved by the RNase III enzyme Drosha to release a 60-70 nt stem-loop intermediate, known as precursor miRNA (pre-miRNA). The hairpin pre-miRNA is actively exported to the cytoplasm, where it is recognized by Dicer and cleaved in a 20-nt miRNA duplex. In the final step of miRNA maturation the functional strand of the small RNA duplex is selectively loaded onto the RNA-induced silencing complex (RISC), whose core components are the Argonaute family proteins. The miRNA then directs the RISC to its target mRNA and represses target gene expression by either destabilizing target mRNAs or repressing their translation. A major determinant of miRNA target recognition is a perfect match in the so-called seed sequence of 6-8 nt at the 5'end of the miRNA (Bartel 2009).

The term "RNA sequences corresponding substantially to miRNAs" as used herein refers to RNA sequences which are sufficient homologue to the RNA sequences of the miRNAs as set forth in Table 1 or the complement sequence thereof, respectively, but allow for derivative molecules of the miRNAs, such as pre- and pri-miRNA or may comprise modified sequences or modified nucleotides or backbone modifications but nonetheless fulfil the criteria that i) if the nucleic acid is an antagonist of the miRNA, the nucleic acid binds (or hybridizes) sufficiently strongly and/or specifically to the miRNA, pre-miRNA or pri-miRNA to thereby inhibit or reduce its activity and ii) if the nucleic acid is an agonist of the miRNA, the nucleic acid mimics or has the biological activity or a significant portion thereof, of the wild-type miRNA.

The term "complement sequence" as used herein - e.g. within the term "RNA sequences corresponding substantially to the RNA sequences of the miRNAs as set forth in Table 1 or the complement sequence thereof' - refers to the conversion into the reverse complement counterpart of the original nucleotide sequence.

The term "more differentiated cells" as used herein refers to cells which stem from hematopoietic stem cells but which have not the primitive status of the CD133⁺ cells themselves anymore, e.g. CD34⁺CD133⁻ cells are more differentiated than CD133⁺ cells.

The term "modulation of cells" or "modulation the differentiation of cells" as used herein refers to any change or prevention of change of the status quo of a cell triggered by an external stimulus. External stimuli can be miRNA sequences like miRNA mimics or AMOs. The modification triggered by the external stimulus may be the prevention of change of the status quo, i.e. the maintenance of the status quo, e.g. the status quo of a primitive CD133⁺ cell and/or progenitor cells. Especially in vitro primitive CD133⁺ cells and/or progenitor cells tend to differentiate to more differentiated cells. The prevention of this differentiation is a modulation of the cell triggered by an external stimulus. Other kinds of modulation are the proliferation of cells, e.g. primitive CD133⁺ cells and/or progenitor cells triggered by an external stimulus, the de-differentiation or reprogramming of more differentiated cells to primitive CD133⁺ cells and/or progenitor cells triggered by an external stimulus, the direct differentiation of primitive CD133⁺ cells and/or progenitor cells to more differentiated cells triggered by an external stimulus, the preventing of apoptosis of the cells triggered by an external stimulus.

The term "homing" as used herein refers to the specific movement or migration of stem cells to the target tissue where they lodge at least transiently by activation of adhesion interactions.

The term "engraftment" as used herein refers to the production of more stem cells by symmetrical divisions and production of a large number of progenitors and differentiated cell types after unique homing to the target tissue.

The term dedifferentiation as used herein refers to the process where more differentiated cells are reverted to more primitive cells, e.g. to CD133⁺ cells and/or progenitor cells.

The term reprogramming as used herein refers to the process where differentiated cells are reverted to pluripotent cells.

The term "miRNA mimics" as used herein refers to small, single or double stranded, chemically-synthetic and optimized nucleic acids designed to mimic endogenous mature microRNA molecules in cells.

The term "AMO" as used herein is the abbreviation for anti-miRNA oligonucleotide and refers to a class of chemically engineered oligonucleotides. AMOs are used to silence endogenous microRNA An AMO is a small synthetic RNA that is reverse complementary to the specific miRNA target. Usually, AMOs have some sort of modification to make it more resistant to degradation.

The term "effective amount" as used herein refers to an amount sufficient to effect benefical or desirable biological and/or clinical results.

The terms "expression vector" as used herein refer to a nucleic acid construct, generated recombinantly or synthetically, bearing a series of specified nucleic acid elements that enable transcription of a particular gene in a host cell. Typically, gene expression is placed under the control of certain regulatory elements, such as constitutive or inducible promoters.

The miRNAs significantly differentially expressed in CD133⁺ cells can be used for in vitro expansion, e.g. transfection of miRNAs higher expressed in CD133⁺ cells maintains the primitive phenotype during in vitro cultivation of CD133⁺ cells.

In one embodiment, a method may comprise contacting hematopoietic stem and progenitor cells with one or more RNA sequences corresponding substantially to the RNA sequences of the microRNAs as set forth in Table 1 or the complement sequence thereof and thereby modulate these cells.

In one embodiment, a method may comprise contacting CD133⁺ cells and/or progenitor cells with one or more RNA sequences corresponding substantially to the RNA sequences of the microRNAs as set forth in Table 1 or the complement sequence thereof and wherein the modulation is the proliferation of said cells and wherein said cells maintain their self-renewal capability.

In one embodiment, a method may comprise contacting CD133⁺ cells and/or progenitor cells with one or more RNA sequences corresponding substantially to the RNA sequences of the microRNAs as set forth in Table 1 or the complement sequence thereof and wherein the modulation is the proliferation of said cells and wherein said cells maintain their primitive status.

In one embodiment, a method may comprise contacting CD133⁺ cells and/or progenitor cells with one or more RNA sequences corresponding substantially to the RNA sequences of the microRNAs as set forth in Table 1 or the complement sequence thereof for the aim of maintenance and proliferation of the primitive status of these cells.

In one embodiment, a method may comprise contacting CD133⁺ cells and/or progenitor cells with one or more RNA sequences corresponding substantially to the RNA sequences of the microRNAs as set forth in Table 1 or the complement sequence thereof and wherein the modulation is preventing cell death including apoptosis and necrosis of these cells.

In one embodiment, a method may comprise contacting CD133⁺ cells and/or progenitor cells with one or more RNA sequences corresponding substantially to the RNA sequences of the microRNAs as set forth in Table 1 or the complement sequence thereof and wherein the modulation is migration, homing and engraftment of these cells.

In one embodiment, a method may comprise contacting CD133⁺ cells and/or progenitor cells with one or more RNA sequences corresponding substantially to the RNA sequences of the microRNAs as set forth in Table 1 or the complement sequence thereof and wherein the modulation is the direct differentiation of said cells.

In other embodiments, a method may comprise contacting more differentiated cells with one or more RNA sequences corresponding substantially to the RNA sequences of the microRNAs as set forth in Table 1 or the complement sequence thereof and wherein the modulation is the dedifferentiation or reprogramming of said cells and wherein said cells are dedifferentiated or reprogrammed to primitive CD133⁺ cells and/or progenitor cells.

In other embodiments, the methods comprise administering one or more microRNA oligonucleotides selected from miR-10a-, miR-23a-, miR-23b-, miR-24-, miR-29a-, miR-29b-, miR-29c-, miR-99a-, miR-125a-5p-, miR-125b-, miR-142-3p-, miR-142-5p-, miR-146a-, miR-146b-5p-, miR-191-, miR-425-, miR-484- and miR-551b-oligonucleotide to targets cells, i.e. CD133⁺ cells and/or progenitor cells or cells which should be dedifferentiated to CD133⁺ cells and/or progenitor cells.

In other embodiments, the methods comprise administering one or more microRNA expression vectors selected from the group consisting of miR-10a-, miR-23a-, miR-23b-, miR-24-, miR-29a-, miR-29b-, miR-29c-, miR-99a-, miR-125a-5p-, miR-125b-, miR-142-3p-, miR-142-5p-, miR-146a-, miR-146b-5p-, miR-191-, miR-425-, miR-484- and miR-551b-expressing vector to targets cells, i.e. CD133⁺ cells and/or progenitor cells or cells which should be dedifferentiated to CD133⁺ cells and/or progenitor cells such that one or more of these miRNAs are expressed in target cells.

In further embodiments, the methods comprise administering one or more anti-miRNA oligonucleotides (AMOs) selected from the group consisting of antisense-miR-10a-, -miR-23a, -miR-23b, -miR-24, -miR-29a, -miR-29b, -miR-29c, -miR-99a, -miR-125a-5p, -miR-125b, -miR-142-3p, -miR-142-5p, -miR-146a, -miR-146b-5p, -miR-191, -miR-425, -miR-484 and -miR-551b or other molecules that interfere with these microRNA expressions or activities to targets cells, i.e. CD133⁺ cells and/or progenitor cells or cells which should be dedifferentiated to CD133⁺ cells and/or progenitor cells.

In yet another embodiment of the invention, any cell culture medium known in the art of cell culturing (Lindl, Gstraunthaler, Zell- und Gewebekultur, 6. edition, 2008) can be used for adding one or more RNA sequences corresponding substantially to the RNA sequences of the microRNAs as set forth in Table 1 or the complement sequence thereof, including one or more of the following media DMEM, HBSS, DPBS, RPMI, Iscove's medium, X-VIVO™, StemSpan (StemCell Technologies, Vancouver, Canada) or CellGro (CellGenix, Freiburg, Germany) optionally supplemented e.g. with fetal calf serum, human serum or serum substitutes or other nutrients or cell stimuli like Cytokines. The media can be classical cell media like the above mentioned media or specialty media for e.g. primary human cell culture (e.g. for endothelia cells, hepatocytes or keratinocytes) or stem cells (e.g. dendritic cell maturation, hematopoietic expansion, keratonocytes, mesenchymal stem cells or T cell expansion). The media may have supplements or reagents well known in the art, e.g. albumins and transport proteins, amino acids and vitamins, antibiotics, attachments factors, growth factors and cytokines, hormones or solubilising agents.

A further embodiment is a cell culture medium comprising one or more RNA sequences corresponding substantially to the RNA sequences of the microRNAs selected from the group consisting of miR-10a-, miR-23a-, miR-23b-, miR-24-, miR-29a-, miR-29b-, miR-29c-, miR-99a-, miR-125a-5p-, miR-125b-, miR-142-3p-, miR-142-5p-, miR-146a-, miR-146b-5p-, miR-191-, miR-425-, miR-484- and miR-551b-oligonucleotide or the complement thereof as compound(s) within the cell culture medium for proliferation and maintenance of the primitive CD133⁺ cells and/or progenitor cells or for dedifferentiating cells to primitive CD133⁺ cells and/or progenitor cells.

A further embodiment is a cell culture medium comprising one or more RNA sequences corresponding substantially to the RNA sequences of the microRNAs selected from the group consisting of miR-10a-, miR-23a-, miR-23b-, miR-24-, miR-29a-, miR-29b-, miR-29c-, miR-99a-, miR-125a-5p-, miR-125b-, miR-142-3p-, miR-142-5p-, miR-146a-, miR-146b-5p-, miR-191-, miR-425-, miR-484- and miR-551b-oligonucleotide or the complement thereof as compound(s) within the cell culture medium for differentiation of primitive CD133⁺ cells and/or progenitor cells to more differentiated cells.

One embodiment of the invention is the use of one or more RNA sequences corresponding substantially to the RNA sequences of the miRNAs selected from the group consisting of miR-10a, miR-23a, miR-23b, miR-24, miR-29a, miR-29b, miR-29c, miR-99a, miR-125a-5p, miR-125b, miR-142-3p, miR-142-5p, miR-146a, miR-146b-5p, miR-191, miR-425, miR-484, miR-551b or the complement sequence thereof for the modulation of cells and wherein the cells are CD133⁺ cells and/or progenitor cells and wherein the modulation is the proliferation of said cells and wherein said cells maintain their self-renewal capability.

One embodiment of the invention is the use of one or more RNA sequences corresponding substantially to the RNA sequences of the miRNAs selected from the group consisting of miR-10a, miR-23a, miR-23b, miR-24, miR-29a, miR-29b, miR-29c, miR-99a, miR-125a-5p, miR-125b, miR-142-3p, miR-142-5p, miR-146a, miR-146b-5p, miR-191, miR-425, miR-484, miR-551b or the complement sequence thereof for the modulation of cells and wherein the cells are more differentiated cells and wherein the modulation is the dedifferentiation or reprogramming of said cells and wherein said cells are dedifferentiated or reprogrammed to primitive CD133⁺ cells and/or progenitor cells.

One embodiment of the invention is the use of one or more RNA sequences corresponding substantially to the RNA sequences of the miRNAs selected from the group consisting of miR-10a, miR-23a, miR-23b, miR-24, miR-29a, miR-29b, miR-29c, miR-99a, miR-125a-5p, miR-125b, miR-142-3p, miR-142-5p, miR-146a, miR-146b-5p, miR-191, miR-425, miR-484, miR-551b or the complement sequence thereof for the modulation of cells and wherein the cells are CD133⁺ cells and/or progenitor cells and wherein the modulation is the direct differentiation of said cells.

### Brief Description of the drawings

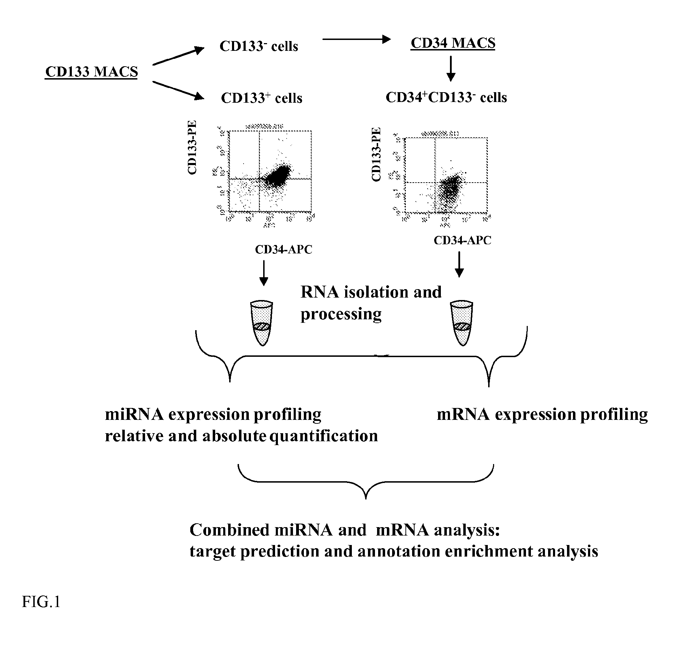

FIG. 1. Experimental procedure for miRNA expression analysis in HSCs and HPCs.

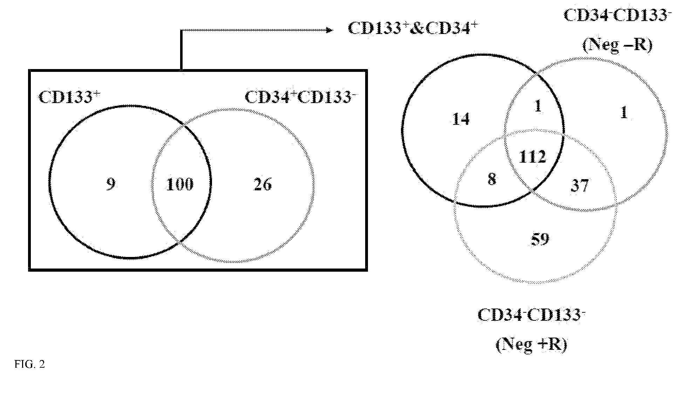

Fig. 2. Number of miRNAs expressed in the different hematopoietic subpopulations. Neg +R: CD34⁻CD133⁻;with red blood cells; Neg -R: CD34⁻CD133⁻ without red blood cells.

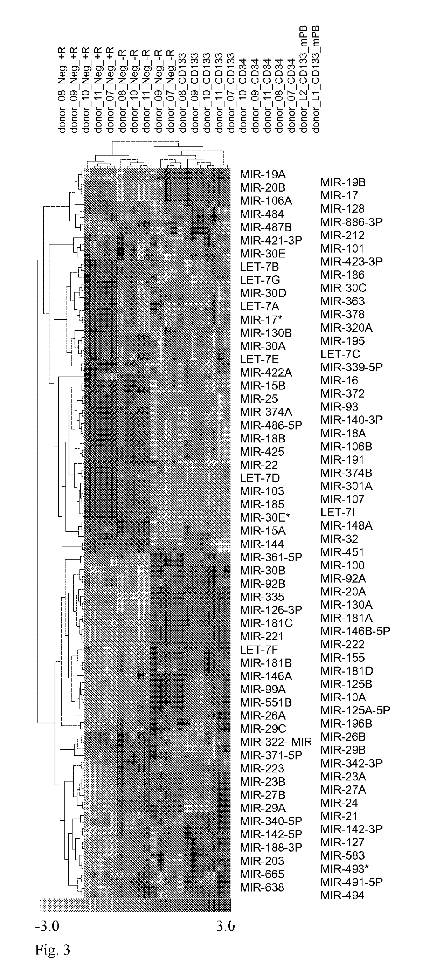

FIG. 3. Average linkage cluster of miRNAs expressed in CD133⁺, CD34⁺CD133⁻ and CD34⁻CD133⁻ (Neg) cells. Neg +R: CD34⁻CD133⁻ with red blood cells; Neg_-R: CD34⁻CD133⁻ without red blood cells. Log2-transformed expression ratios are indicated from -3.0 (grey) to 3.0 (black).

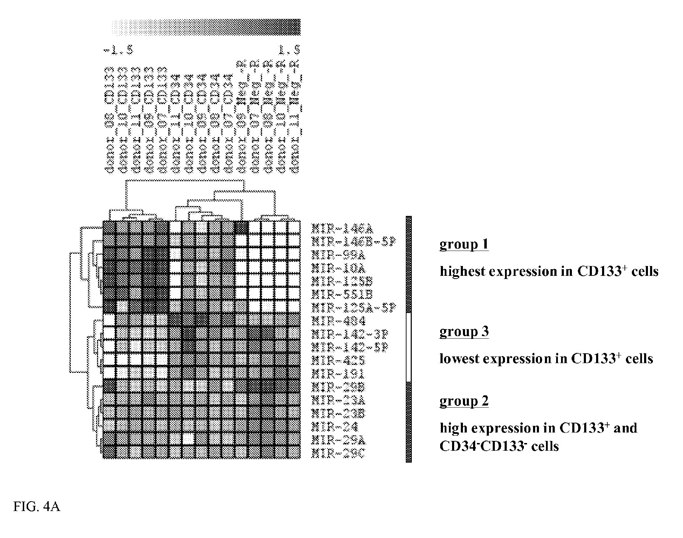

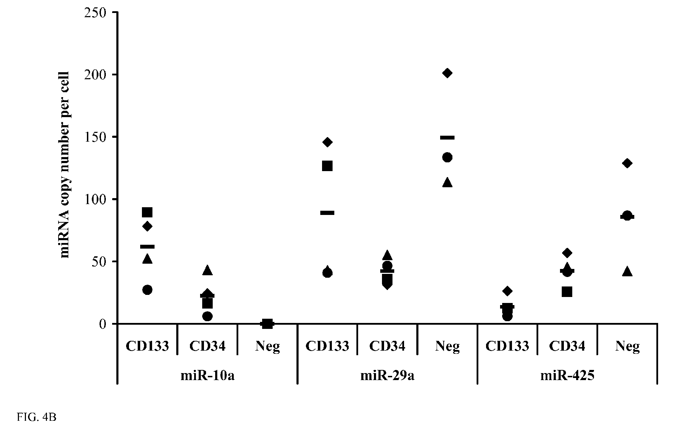

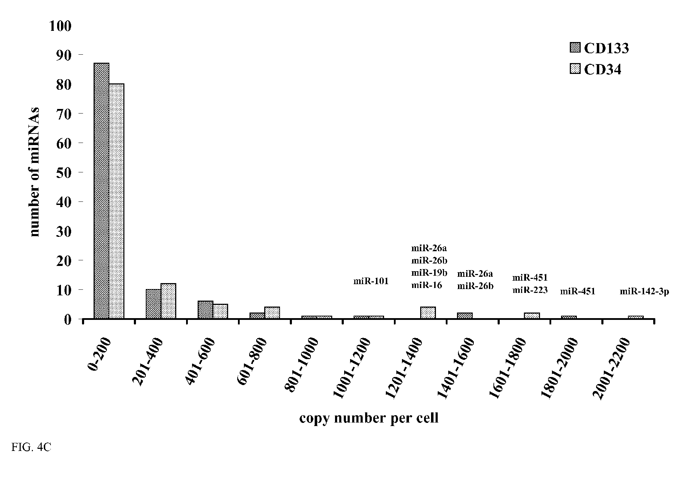

FIG. 4. Relative and absolute expression of miRNAs in CD133⁺ cells (A) Cluster analysis of miRNAs identified by discriminatory gene analysis of CD133⁺ cells compared to CD34⁺CD133⁻ cells. (B) MicroRNA copy numbers prepared from four different donors (donor no. 7, 8, 10 and 11). (C) Histogram displaying the number of miRNAs in distinct copy number intervals, e.g. 87 of the miRNAs detected in CD133⁺ cells were present with less than 200 copy numbers per cell and 10 miRNAs with copy numbers between 201 and 400.

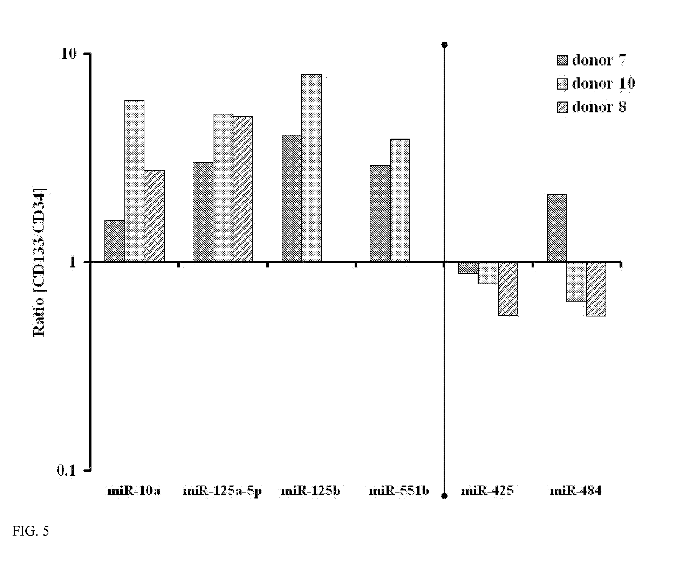

FIG. 5. Validation of microarray data via qRT-PCR. The expression levels of six miRNAs were also determined by qRT-PCR using the miScript system for two (miR-125b, miR-551b) or three donors. RN5S1 was used for normalization.

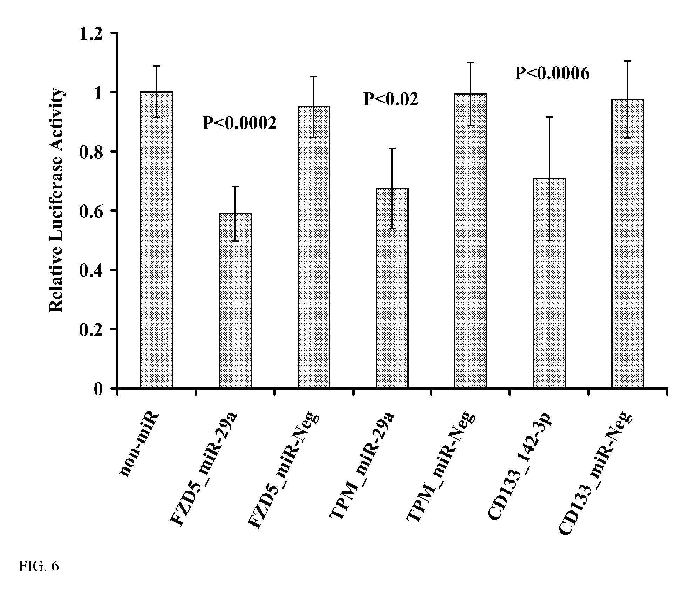

FIG. 6. Verification of miRNA-mRNA interactions via luciferase assay. Cotransfection of pMIR-Luc-FZD5 and 100 nM miR-29a decreased 41% of the luciferase activity in comparison to the blank control. Cotransfection of pMIR-Luc-TPM and miR-29a decreased the luciferase activity by 33% of, pMIR-Luc-CD133 and miR-142-3p by 30%. For each cotransfection 300 ng vector and 50 nM (miR-142-3p) or 100 nM (miR-29a) oligonucleotide were used. Relative luciferase activity represents firefly luciferase activity normalized versus renilla acitivity. Data are representative of at least five independent experiments. miR-Neg: oligonucleotide designed to serve as negative control.

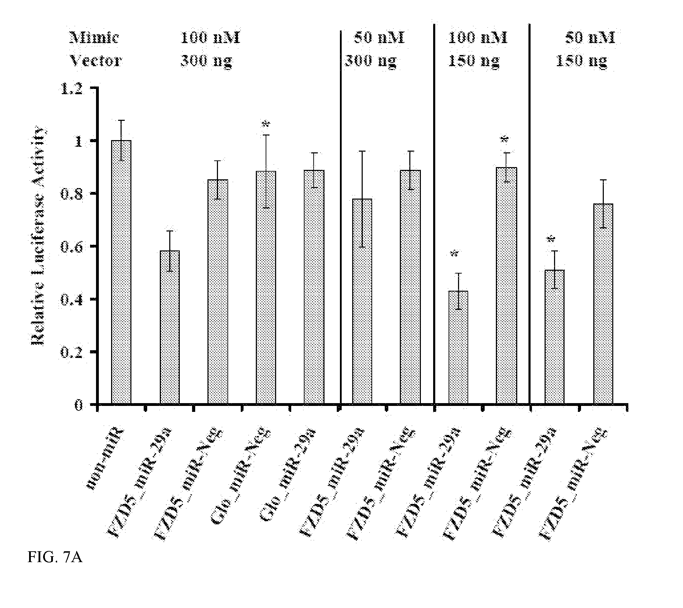

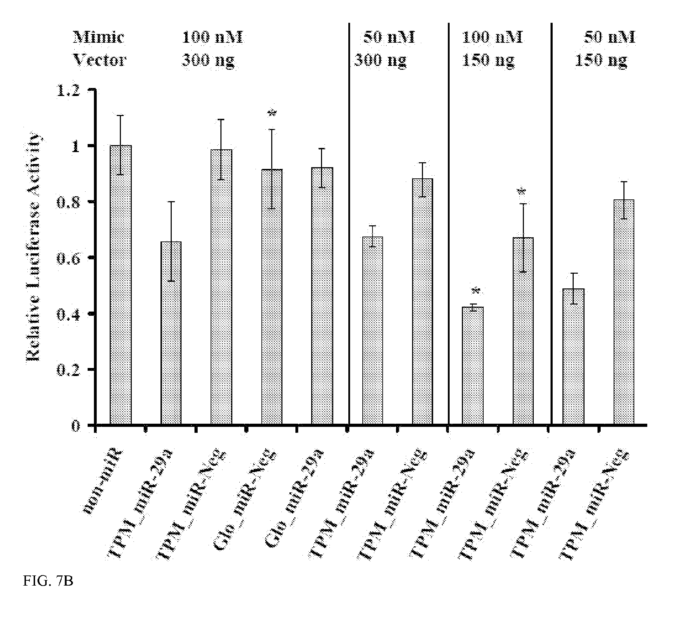

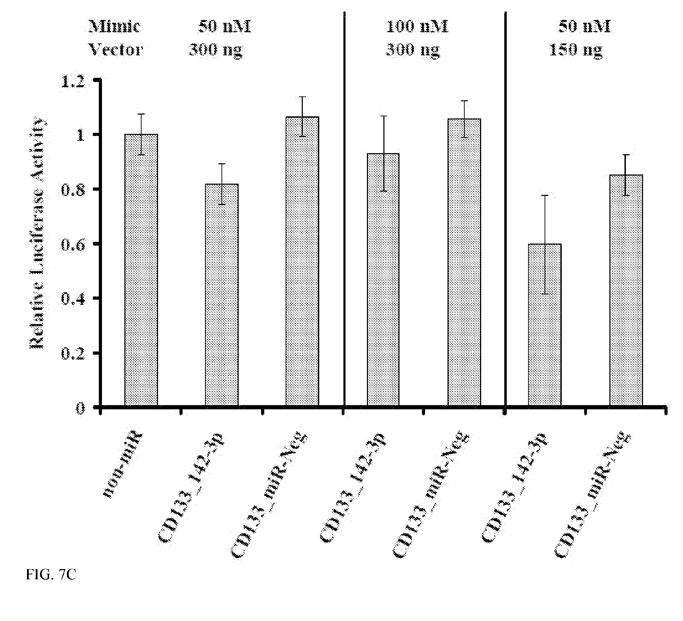

FIG. 7. Verification of miRNA-mRNA interactions using different mimic (oligonucleotides) and vector amounts. (A) Cotransfection of pMIR-Luc-FZD5 and miR-29a. (B) Cotransfection of pMIR-Luc-TPM and miR-29a. (C) Cotransfection of pMIR-Luc-CD133 and miR-142-3p Relative luciferase activity represents firefly luciferase activity normalized versus renilla acitivity. Data are representative of at least 3 independent experiments. Data with an asterisk (*) were performed in duplicates.

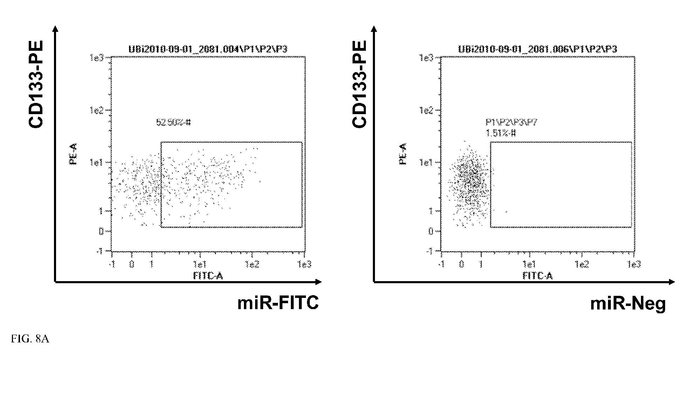

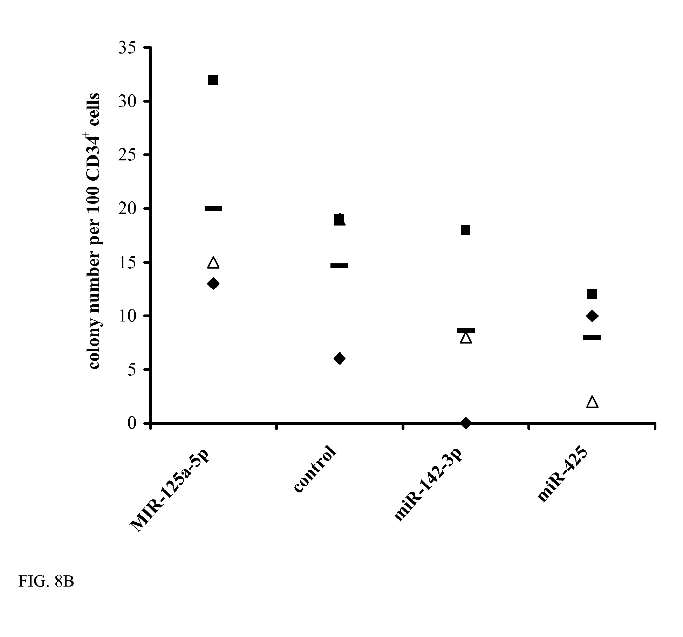

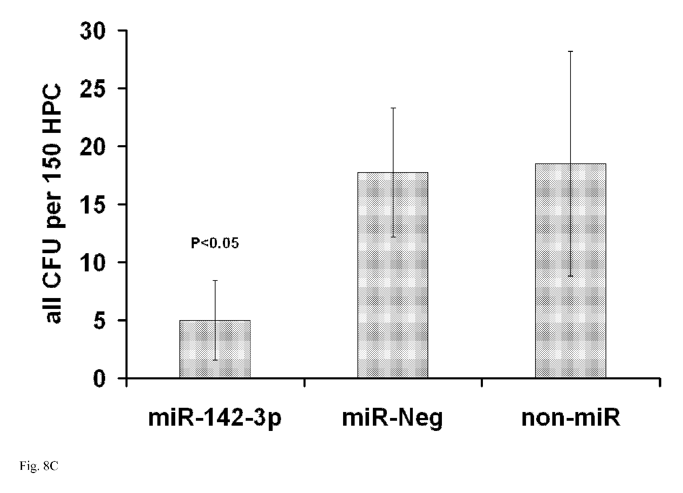

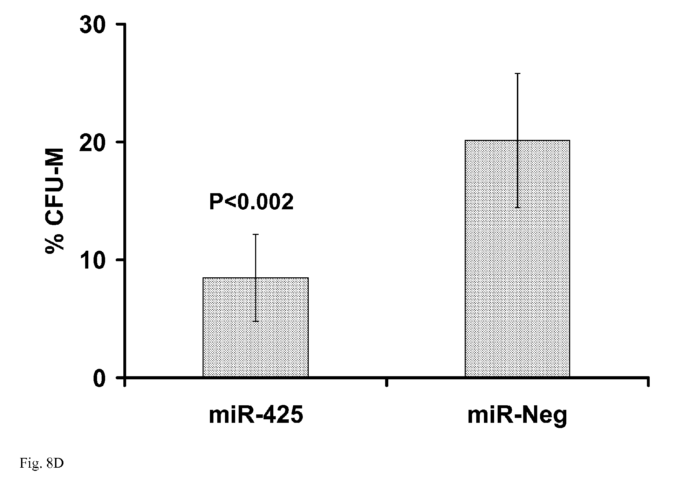

FIG 8. Transfection of CD133⁺ cells with different miRNA mimics. (A) Transfection of FITC labeled miRNA mimic. (B) HSC-CFU assay for cells transfected with miRNAs. Cells were seeded after transfection and after several days of cultivation CFU colonies were classified and counted. Three different donors are shown. (C) Effect of miR-142-3p on CD133⁺ cells. The overall colony forming ability was decreased by 71% (n=4; P<0.05). (D) CD133⁺ cells were transfected with miR-425 or control (miR-Neg). The percentage of CFU-M (colony forming unit - macrophage) decreased from 20% to 8.5%.

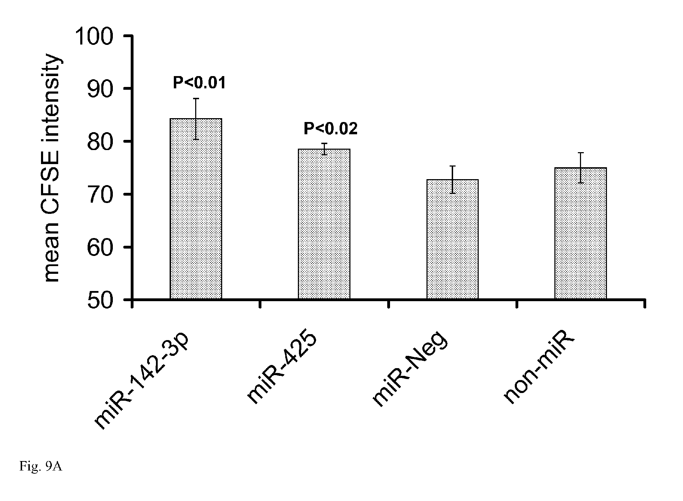

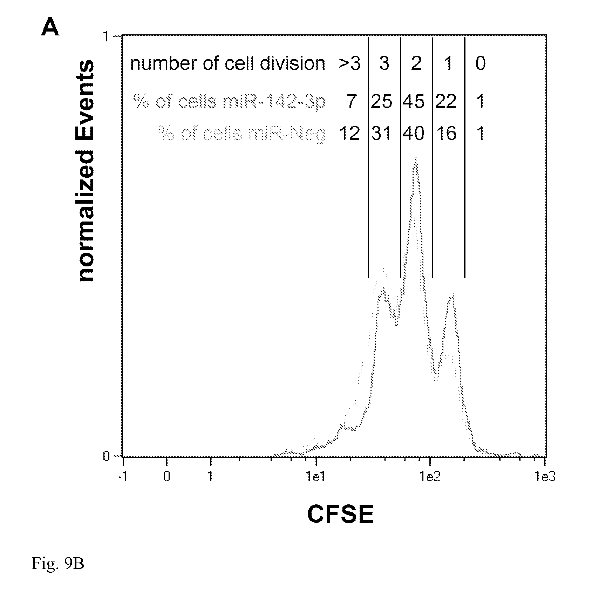

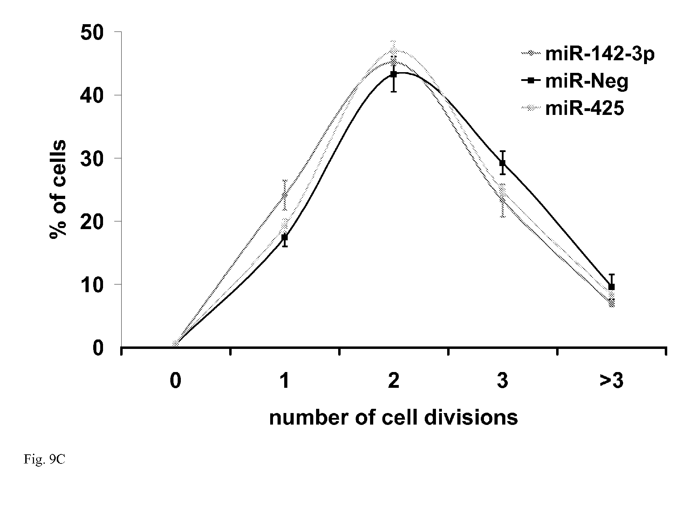

FIG 9. Influence of miR-142-3p and miR-425 on proliferation of CD133⁺ cells transfected with miRNAs. CD133⁺ cells were stained with CFSE, transfected with miRNAs and cultured for 6 days. (A) Mean CFSE intensity of CD133⁺ cells was higher for cells transfected with miR-142-3p and miR-425 as compared to the negative controls. Presented significance values were determined in relation to miR-Neg (n=4). (B) The number of cell divisions and the percentage of cells in each division is indicated. CD133⁺ cells transfected with miR-142-3p contained a lower proportion of cells that passed through more than two cell divisions compared to cells transfected with miR-Neg. One representative histogram is shown. (C) Proportion of CD133⁺ cells in each cell division (n=4).

### Detailed Description of the invention

The inventors found that the specific miRNAs as set forth in Table 1 or the complement sequence thereof can be used to modulate CD133⁺ hematopoietic stem cells by preventing the differentiation, allowing direct differentiation or dedifferentiation.

miRNAs have been shown to play an important role in many different cellular, developmental, and physiological processes including hematopoiesis. The inventors characterized the role of miRNAs in early hematopoiesis and isolated subpopulations of hematopoietic stem and progenitor cells expressing CD34 and/or CD133.

Semiquantitative and quantitative miRNA profiling using a miRNA microarray platform (miRXplore™) revealed that a number of miRNAs were significantly differentially expressed between CD133⁺ and CD34⁺CD133⁻ cells (Table 1). These results were validated via qRT-PCR.

The targets of the significantly differentially expressed miRNAs were predicted by using bioinformatic tools. Luciferase reporter based assays were used to validate some of the predicted miRNA-mRNA interactions. HSC-CFU assays were performed to analyze the significantly differentially expressed miRNAs as a potential tool for *in vitro* expansion of HSCs.

The microRNAs as set forth in Table 1 were significantly differentially expressed between CD133⁺ and CD34⁺CD133⁻ cells. Out of these 18 miRNAs 13 miRNAs (group 1 and 2) were significantly higher expressed in CD133⁺ compared to CD34⁺CD133⁻ cells. These 13 miRNAs could be divided into one group of miRNAs that were not expressed in CD34⁻CD133⁻ cells (group1: miR-10a, miR-99a, miR-125a-5p, miR125b, miR146a, miR-146b-5p, miR-551b) and another group where the expression in CD34⁻CD133⁻ cells was even higher than in CD133⁺ cells (group 2: miR-23a, miR-23b, miR-24, miR-29a, miR-29b, miR-29c). Five miRNAs (group 3: miR-191, miR-142-3p, miR142-5p, miR-425, miR-484) were significantly lower expressed in CD133⁺ cells as in CD34⁺CD133⁻ cells.

**Table1. miRNAs significantly differentially expressed in CD133⁺ cells compared to CD34⁺CD133⁻ miRBase accession: MIMAT accession number. Group: definition to group 1, 2 and 3 in description.**

| **miRNA** | **miRBase accession** | **Group** |
|---|---|---|
| miR-10a | 0000253 | 1 |
| miR-23a | 0000078 | 2 |
| miR-23b | 0000418 | 2 |
| miR-24 | 0000080 | 2 |
| miR-29a | 0000086 | 2 |
| miR-29b | 0000100 | 2 |
| miR-29c | 0000681 | 2 |
| miR-99a | 0000097 | 1 |
| miR-125a-5p | 0000443 | 1 |
| miR-125b | 0000423 | 1 |
| miR-142-3p | 0000434 | 3 |
| miR-142-5p | 0000433 | 3 |
| miR-146a | 0000449 | 1 |
| miR-146b-5p | 0002809 | 1 |
| miR-191 | 0000440 | 3 |
| miR-425 | 0003393 | 3 |
| miR-484 | 0002174 | 3 |
| miR-551b | 0003233 | 1 |

In order to further analyze the role of the differentially expressed miRNAs in CD133⁺ stem cells, standard bioinformatic tools were used to predict possible targets of the differentially expressed miRNAs. Luciferase assays were carried out to validate the predicted miRNA-mRNA interactions.

The luciferase assays suggested that FZD5 and TPM1 are controlled by miR-29a. Another interaction that was validated via luciferase assay was the binding of miR-142-3p, being lower expressed in CD133⁺ cells than in CD34⁺CD133⁻ cells, to the CD133 mRNA (Entrez ID: 8842). A regulation of CD133 expression via miRNAs might be highly relevant for the in vitro expansion of CD133⁺ cells.

The influence of some miRNAs - significantly differentially expressed in CD133⁺ cells compared to CD34⁺CD133⁻ cells - on the cultivation of CD133⁺ cells was tested. The CD133⁺ cells were cultivated and transfected with the miRNA mimics or anti-miRNA oligonucleotids (AMO) of the miRNAs as set forth in Table 1. Cells were seeded after transfection in HSC-CFU medium. After several days of cultivation the colony-forming ability was analyzed.

In general, the number of colonies was higher for cells transfected with mimics of miRNAs selected of the group 1 or 2 of miRNAs as specified in Table 1 applied as single miRNAs or in several combinations of miRNAs compared to non-transfected cells. This was in accordance with the assumption that overexpression of miRNAs - already highly expressed in CD133⁺ cells - maintained the cells in the primitive status of CD133⁺ cells, leading to a higher colony-forming ability.

In general, the number of colonies was lower for cells transfected with AMOs of miRNAs selected of the group 1 or 2 of miRNAs as specified in Table 1 applied as single miRNAs or in several combinations of miRNAs compared to non-transfected cells. This was in accordance with the assumption that down-regulation of miRNAs - normally highly expressed in CD133⁺ cells - pushed the cells towards CD133⁻ cells, leading to a lower colony-forming ability.

In general, the number of colonies was higher for cells transfected with AMOs of miRNAs selected of the group 3 of miRNAs as specified in Table 1 applied as single miRNAs or in several combinations of miRNAs compared to non-transfected cells. This was in accordance with the assumption that down-regulation of miRNAs - already weakly expressed in CD133⁺ cells - maintained the cells in the primitive status of CD133⁺ cells, leading to a higher colony-forming ability.

In general, the number of colonies was lower for cells transfected with mimics of miRNAs selected of the group 3 of miRNAs as specified in Table 1 applied as single miRNAs or in several combinations of miRNAs compared to non-transfected cells. This was in accordance with the assumption that overexpression of miRNAs - lower expressed in CD133⁺ cells - pushed the cells towards CD133⁻ cells, leading to a lower colony-forming ability.

In one embodiment of the invention, CD133⁺ cell modulation can be modulated by modulating the level(s) of one or more RNA sequences corresponding substantially to the RNA sequences of the microRNAs as set forth in Table 1 or the complement sequence thereof in the target cell, i.e. CD133⁺ cells and/or progenitor cells or cells which should be dedifferentiated to CD133⁺ cells.

Upregulation of one or more of these miRNAs in target organs, tissues and/or cells can be accomplished by, e.g. administering to the target either synthetic miRNA(s) such as a miRNA-oligonucleotide, so-called miRNA mimics, or expression vectors that express miRNA(s). Downregulation of one or a combination of these miRNAs in target organs, tissues and/or cells can be accomplished by, e.g. administering to the target anti-miRNA(s), such as by administering synthetic antisense miRNA(s), so-called AMOs, expression vectors that express antisense miRNA(s) or administering one or more other molecules that interfere with miRNA(s) expression or activity.

In another embodiment of the invention proliferation and self-renewal capability of CD133⁺ cells can be upregulated by administering one or more RNA sequences corresponding substantially to the RNA sequences of the microRNA(s) - miRNA mimics - selected from the group 1 of miRNAs (miR-10a, miR-99a, miR-125a-5p, miR125b, miR146a, miR-146b-5p, miR-551b) and group 2 of miRNAs (miR-23a, miR-23b, miR-24, miR-29a, miR-29b, miR-29c) as set forth in Table 1 to CD133⁺ cells and/or progenitor cells.

In further embodiments of the invention proliferation and self-renewal capability of CD133⁺ cells and/or progenitor cells can be upregulated by administering one or more anti-miRNAs-AMOs - or one or more other molecules that interfere with the miRNA expression selected from the group 3 of miRNAs as set forth in Table 1 (miR-191, miR-142-3p, miR142-5p, miR-425, miR-484).

In another embodiment of the invention direct differentiation of CD133⁺ cells and/or progenitor cells to more differentiated cells can be upregulated by administering one or more AMO or one or more other molecules that interfere with the miRNA expression selected from the group 1 of miRNAs (miR-10a, miR-99a, miR-125a-5p, miR125b, miR146a, miR-146b-5p, miR-551b) and group 2 of miRNAs (miR-23a, miR-23b, miR-24, miR-29a, miR-29b, miR-29c) as set forth in Table 1 to CD133⁺ cells.

In one embodiment of the invention direct differentiation of CD133⁺ cells and/or progenitor cells to more differentiated cells can be upregulated by administering one or more RNA sequences corresponding substantially to the RNA sequences of the microRNAs selected from the group 3 of miRNAs as set forth in Table 1 (miR-191, miR-142-3p, miR142-5p, miR-425, miR-484).

In one embodiment of the invention dedifferentiating capability of more differentiated cells to primitive CD133⁺ cells and/or progenitor cells can be upregulated by administering one or more RNA sequences corresponding substantially to the RNA sequences of the microRNAs selected from the group 1 of miRNAs (miR-10a, miR-99a, miR-125a-5p, miR125b, miR146a, miR-146b-5p, miR-551b) and group 2 of miRNAs (miR-23a, miR-23b, miR-24, miR-29a, miR-29b, miR-29c) as set forth in Table 1 to the more differentiated cells.

In another embodiment of the invention dedifferentiating capability of more differentiated cells to primitive CD133⁺ cells and/or progenitor cells can be upregulated by administering one or more AMO or one or more other molecule that interfere with the miRNA expression selected from the group 3 of miRNAs as set forth in Table 1 (miR-191, miR-142-3p, miR142-5p, miR-425, miR-484).

A method may comprise contacting a target cell with one or more RNA sequences corresponding substantially to the RNA sequences of the miRNAs as set forth in Table 1 or the complement sequence thereof. Contacting may comprise providing one or more of these RNA sequences to said target cell, or providing an expression construct encoding said RNA sequence to said target cell. The cell may be located in an animal subject, such as a human, or the cell may be contacted in vitro, wherein the method may comprise further culturing of said cell with or without reintroduction into an animal.

Enriching or expanding a population of cells may be conducted in vitro, ex vivo or in vivo. When expanding a population of cells in vitro, the initial population of cells may be from a cell line, including embryonic or cancer stem cell lines, or from a subject. An initial population of cells may comprise multiple cell types, or alternatively, it may consist essentially of only one cell type. Where an initial population of cells comprises different types of cells, it may first be enriched in certain types of cells by using techniques known in the art, by immunoaffinity (e.g. FACS, MACS). Accordingly, an initial population of cells may be a population of cells that is enriched in certain types of cells. In one example, an initial population of cells is obtained from the peripheral blood or bone marrow of a subject and the population of cells has been enriched in CD133⁺ cells.

In one embodiment of the invention, an initial population of cells is obtained from a subject; the initial population of cells is enriched or expanded into one or more particular cell types to obtain an enriched or expanded population of cells; and the enriched or expanded population of cells according to the invention is administered to a subject, which may be the same subject from whom the initial population of cells was obtained or a different subject. The cells may also be additionally purified, enriched or expanded by other methods, e.g., based on the presence of certain surface markers, e.g. CD133.

In one embodiment of the invention, a population of cells is enriched or expanded in vivo. A method may comprise administering to a subject in need thereof a therapeutically effective amount of one or more RNA sequences corresponding substantially to the RNA sequences of the miRNAs as set forth in Table 1 or the complement sequence thereof to thereby enrich or expand a population of cells in the subject. An agent may be administered locally or systemically. In one embodiment, for expanding or enriching hematopoietic cells in certain types of cells, an agent is administered into the bone marrow. For enriching or expanding a population of cells in the blood, an agent may be administered directly into the blood. An agent may comprise or be comprised in a delivery vehicle that allows targeting to particular cell types. Such targeting may be based on cell surface molecules of the target population of cells.

Also provided herein are isolated, purified, expanded and/or enriched populations of cells that are obtained using one or more of the methods described herein for modulating the differentiation of cells. A population of cells may at least about 50%, 60%, 70%, 80%, 90%, 95%, 97%, 98%, 99% or 100% enriched in one or more type or lineage of cells, e.g. a CD133⁺ cell population.

Populations of cells may also comprise cells comprising one or more heterologous nucleic acid encoding or identical to one or more miRNAs as set forth in Table 1. Populations of cells may also be comprised in a pharmaceutical composition, e.g., for administration of the cells to a subject, e.g., in stem cell therapy. Populations of cells and compositions comprising such may also be present in a delivery vehicle, such as a liposome, a viral particle or a nanoparticle.

In one embodiment of the invention, expanded populations of early stem cells, e.g. CD133⁺ cells, may be used for the production of cells of one or more lineage(s), which may then be transplanted into a subject.

Alternatively, expanded populations of cells, e.g., undifferentiated cells like CD133⁺ cells, are administered to a subject, e.g., for stem cell therapy.

It is within the meaning of the invention that the sequences of the miRNAs miR-10a, miR-23a, miR-23b, miR-24, miR-29a, miR-29b, miR-29c, miR-99a, miR-125a-5p, miR-125b, miR-142-3p, miR-142-5p, miR-146a, miR-146b-5p, miR-191, miR-425, miR-484, miR-551b or the complement sequences thereof may also comprise a sequence that is the corresponding sequence of the respective pre-miRNA or pri-miRNA. When referring to "comprise a sequence", it is understood that in certain embodiments, "consisting essentially of" or "consisting of" are also included. For example, in certain embodiments, a nucleic acid comprises, consists essentially of or consists of a nucleotide sequence that is at least about 50%, 60%, 70%, 80%, 85%, 90%, 95%, 96%, 97%, 98%, 99% or 100% to that of the miRNA, pre-miRNA or pri-miRNA or the complement thereof. In certain embodiments, a nucleic acid comprises a nucleotide sequence that differs (by substitution, addition or deletion) from that of a miRNA, pre-miRNA or pri-miRNA in about 1, 2, 3, 4, 5, 6, 7, 8, 9, 10 or more nucleotides. In embodiments in which it is desired to inhibit the differentiation of a stem cell or a stem-progenitor cell, a nucleic acid comprising a nucleotide sequence that is identical to or homologous to that of all or a portion of the miRNA, pre-miRNA or pri-miRNA may be used. In embodiments in which it is desired to stimulate the differentiation of a stem cell, a nucleic acid comprising a nucleotide sequence that is identical to or homologous to that of the complement of all or a portion of the miRNA, pre-miRNA or pri-miRNA may be used. Generally, when referring to targeting, modulating, inhibiting or stimulating a miRNA, it is understood that this may include affecting related or derivative molecules of the miRNA, such as pre- and pri-miRNAs. A difference in nucleotide sequence is permitted provided that i) if the nucleic acid is an antagonist of the miRNA, the nucleic acid binds (or hybridizes) sufficiently strongly and/or specifically to the miRNA, pre-miRNA or pri-miRNA to thereby inhibit or reduce its activity and ii) if the nucleic acid is an agonist of the miRNA, the nucleic acid mimics or has the biological activity or a significant portion thereof, of the wild-type miRNA.

Nucleic acids for modulating the level or activity of miRNAs may, in addition to a sequence that is identical or homologous to that of the miRNA or the complement sequence thereof, also comprise additional, unrelated nucleotides, provided that they do not interfere with the mimicking or inhibition, respectively, of the miRNA. They may comprise, e.g., about 1, 2, 3, 4, 5, 6, 7, 8, 9, 10 or more nucleotides that are located either 5' and/or 3' (or internal) to the sequence for modulating the level or activity of the miRNA.

Delivery of oligonucleotides and/or expression vectors to a target cell can be achieved in a variety of ways. In some embodiments, a transfection agent is used. A transfection agent, or transfection reagent or delivery vehicle, is a compound or compounds that bind(s) to or complex(es) with oligonucleotides and polynucleotides, and enhances their entry into cells. Examples of transfection reagents include, but are not limited to, cationic liposomes and lipids, polyamines, calcium phosphate precipitates, polycations, histone proteins, polyethylenimine, polylysine, and polyampholyte complexes. Transfection reagents are well known in the art. Transfection reagents suitable for delivery of miRNA are e.g. HiPerfect (Qiagen) and NeoFX.^{™} transfection agent (Ambion), which can be used to transfect a variety of cell types with miRNA. miRNAs can be readily electroporated into primary cells without inducing significant cell death. In addition, miRNAs can be transfected at different concentrations.

Transfection agents may also be used to associate functional groups with a polynucleotide. Functional groups can include cell targeting moieties, cell receptor ligands, nuclear localization signals, compounds that enhance release of contents from endosomes or other intracellular vesicles (such as membrane active compounds), and other compounds that alter the behavior or interactions of the compound or complex to which they are attached (interaction modifiers).

In other embodiments of the invention, the oligonucleotides enter into cells without a transfection agent. This is preferred for cell culture media using oligonucleotides, e.g. miRNA mimics or AMOs, as one component of the cell culture medium. In case of use of miRNA mimics and/or AMOs in the cell culture medium these RNA sequences may be stabilized to prevent fast degradation.

To increase stability and/or optimize delivery of miRNAs as set forth in table 1or complement thereof, modified nucleotides or backbone modifications can be utilized. For example, modified nucleotides may include: linked nuclear acid (LNA), 2 -0-Me nucleotides, 2'-O-methoxyethyl, and T fluoro. Backbone modifications include, for example, phosphorothioate and phosphate. A 3' terminal cholesterol group appears to aid delivery of oligonucleotides like miRNAs to cells. These modifications are reviewed in Horwich and Zamore, Nat Protoc (2008) 3(10):1537-1549, which is herewith incorporated by reference.

In other embodiments of the invention oligonucleotides with incorporated caged nucleotides are used e.g. by attaching light removable protection groups to miRNA mimics and/or AMO.

The following examples are provided to illustrate the invention and are non-limiting to the scope of the claims.

### Examples

### Example 1: Methods

### Cells and cell sorting

HSCs were isolated from human bone marrow following written consent from the local Ethics Committee. The mean donor age was 79 years. CD133⁺ bone marrow cells were enriched with the CliniMACS CD133 Reagent (clone AC133, Miltenyi Biotec) using the CliniMACS Cell Separation System (Miltenyi Biotec, Bergisch Gladbach, Germany). CD34⁺/CD133⁻ cells were isolated by enrichment of CD34⁺ cells with the CliniMACS CD34 Reagent (clone QBEND/10, Miltenyi Biotec) using the negative fraction of the first separation. The mean purity of the viable CD133⁺ cells was 82% ± 10% (n=5) and of the viable CD34⁺CD133⁻ cells 84%± 2% (n=5) as determined by flow cytometric analysis according to ISHAG (Sutherland et al. 1996). CD133(+) cells from mobilized peripheral blood were obtained from AllCells (Berkely, USA).

### RNA sources

Total RNA was isolated from the different hematopoietic subpopulations using miRNeasy Mini (Qiagen). RNA quality was confirmed using an RNA 6000 Pico total RNA kit (Agilent). RINs (RNA Integrity Numbers) were between 8.2 and 9.4. The Universal Reference (UR; miRXplore™ Universal Reference, Miltenyi), consisting of a pool of 954 miRNAs, with each individual oligoribonucleotide having a final concentration of 5 fmol/µl, has been described in detail (Bissels et al. 2009).

### RNA processing

For miRNA profiling, RNA samples were complemented with miRControl 3 and labeled using the miRCury Power Labelling Kit (Exiqon). Hybridization of miRXplore microarrays was performed as described elsewhere (Bissels et al. 2009) using an automated hybridization instrument (a-Hyb^{™} Hybridization Station, Miltenyi Biotec).

### Microarray Design and Analysis

The miRNA microarray design of the miRXplore microarrays (Miltenyi Biotec) has been described in detail previously (Bissels et al. 2009). The exact configuration of each microarray is deposited at the National Center for Biotechnology Information Gene Expression Omnibus (GEO; accession number GSE22460). Signal processing and quantification of the microarray images was done as described before (Landgraf et al. 2007). Depending on the experimental setup, different ratio calculation and normalization methods were applied using an in house software (miRXplorer) as described previously (Bissels et al. 2009).

For downstream statistical analysis of miRNA array data, ratios were log2 transformed and data imported in TIGR MeV (Saeed et al. 2003). Two dimensional hierarchical clustering (Eisen et al. 1998) was done using Euclidean Distance. The statistical analysis of microarrays was performed using SAM (Tusher et al. 2001) with at least 100 permutations per analysis.

### Quantitative Real-Time PCR (qRT-PCR)

The miScript PCR System (QIAGEN) was used to validate miRNAs detected as differentially expressed on microarrays. The RT-PCR reaction and the real-time PCR were carried out according to the manufacturer's protocol. Data were normalized using RNU6B (MS00014000) or RN5S1(MS00007574). PCR reactions were performed in triplicates and carried out in an ABI Prism 7000 SDS Real Time PCR machine (Applied Biosystems) and analyzed using ABI Prism 7000 System SDS software (version 1.1).

### miRNA target prediction

Prediction of miRNA targets was performed using TargetScan miRDB, PicTar, ElMMo, miRanda and PITA (Bartel 2009).

### Luciferase Assays

60mer DNA oligonucleotides (Metabion) consisting of the test sequence (FZD5: AAAC TACATATGGCCAAGGTCACTTCCGTTTACCTTCATGGTGCTGTTGCCCCCTCCCC; TPM1: AAACTACATATGTGTTGGAAACACAATCAGGTGTGGATTGGTGC TACTTTGAACAAAAC; CD133: AAACTAGCGGCCGCACTTTTTTACACTGAGT TTCTATTTAGACACTACAACATATGGGGTGC) flanked by PmeI, XhoI (overhangs) and NdeI or NotI (internal) restriction sites were cloned downstream of the firefly luciferase gene into the pmirGLO Dual-Luciferase miRNA Target Expression Vector (Promega, Madison, USA) using standard procedures.

HEK293T cells were plated into 96-well plates and cotransfected with the described luciferase reporter construct and the appropriate miRNA precursor (Applied Biosystems) using Attractene (Qiagen). Lumincescence was quantitated 48 hours after transfection by using the Dual Glo Luciferase Assay System (Promega) on a GENios Reader (Tecan).

### Cultivation of CD133⁺ cells

CD133⁺ cells were cultivated in StemSpan serum-free medium (StemCell Technologies, Vancouver, Canada) or in CellGro (CellGenix, Freiburg, Germany) supplemented with STF+I+A: 10 µg/mL heparin (Ratiopharm, Ulm, Germany), 10 ng/mL mouse SCF (R&D Systems, Minneapolis, USA), 20 ng/mL mouse TPO (R&D Systems), 10 ng/mL human FGF-1 (Invitrogen, Frederick, MD), human IGFBP2 (R&D Systems) and human Angpt15 (Abnova, Taipei City, Taiwan). STF supplement is the same without IGFBP2 and Angpt15. Cells were cultured at 37°C in 5% CO2 in U-bottom 96-well plates with 150-200 µl of the indicated medium.

### CFU and CFSE assay

Colony forming unit (CFU) assays were performed to study the developmental potential of CD133⁺ and CD34⁺ cells. For cultivation cells were added to methylcellulose media (HSC-CFU complete with Epo; Miltenyi) and plated in 6-well plates at a final concentration of 100 CD34⁺cells/ml. After incubation for 14 days at 37° C and 5 % CO₂ colonies were classified by their color and morphology using an inverted microscope.

The number of cell divisions was analyzed using carboxyfluorescein diacetate N-succinimidyl ester (CFSE; Sigma-Aldrich) labeling as described by (Walenda et al. 2010). One day after CFSE labeling the CD133⁺ cells were contacted with miRNA mimics or AMO as described below.

### Contacting miRNA with cultivated CD133⁺ cells

CD133⁺ cells from mobilized peripheral blood were thawed, washed and plated at 10⁴ cells per 150 µl in StemSpan + STF (see section 2.4.1) in one well of a U bottom 96-well plate. After one day of cultivation CD133⁺ cells were contacted with double-stranded RNAs that mimic endogenous precursor miRNAs (Applied Biosystems). A FITC labelled control was obtained from Qiagen (AllStars Negative Control siRNA). Contacting miRNAs with the cells was performed either with or without transfection reagent. If contacting was performed with transfection reagent, HiPerfect (Qiagen) was used. Briefly, the transfection mix consisting of miRNA mimics or AMO; HiPerfect and StemSpan was incubated for 10 min and added to the cells.

### Example 2: Relative expression of miRNA in CD133⁺ cells

CD133⁺ cells were magnetically isolated from bone marrow by MACS technology. Subsequently, CD34⁺CD133⁻ cells were isolated using the negative fraction of the first separation (FIG. 1). Besides the CD34⁺CD133⁻ cell population, CD34⁻CD133⁻ cells (the negative fraction of the second separation) were also analyzed and compared to CD133⁺ cells to get a general impression of miRNA expression in bone marrow cells. As the main cell type present in the CD34⁻CD133⁻ fraction are erythrocytes, an additional analysis of CD34⁻CD133⁻ cells after red blood cell (RBC) lysis was performed (Neg -R). Here the main cell population are neutrophilic granulocytes followed by (Terstappen and Levin 1992).

RNA samples from the bone marrow subpopulations - CD133⁺, CD34⁺CD133⁻, CD133⁻CD34⁻ (Neg +R) and CD133⁻CD34⁻ (Neg -R) - of five different donors and additionally two samples of CD133⁺ cells from mobilized leukapheresis (Table 2) were labeled with Cy5 and hybridized versus the Cy3 labeled miRXplore universal reference (UR). The UR is an equimolar pool of 954 synthetic miRNAs of known concentration, that allows direct comparison of miRNA expression across multiple experiments and absolute quantification (Bissels et al. 2009).

The overall number of detected miRNAs (in at least four out of five donors) was 109 in CD133⁺ cells, compared to 126 miRNAs in CD34⁺CD133⁻ cells (FIG. 2), 151 miRNAs in CD133⁻CD34⁻ cells without RBCs (Neg -R), and 216 miRNAs in CD133⁻CD34⁻ cells with RBCs (Neg +R). The diversity of miRNAs was highest in the CD133⁻CD34⁻ cell population including RBCs and lowest in CD133⁺ and CD34⁺CD133⁻ cells.

**Table 2. CliniMACs separation of CD133⁺ and CD34⁺CD133⁻ cells from bone marrow. The RNA was isolated according to the miRNeasy protocol. Purity = overall purity related to all cells; BM = bone marrow; F = female; M = male; oriori = percentage of CD133⁺ cells in original fraction before sample processing.**

| | Donor | | | CD133 Separation | | | | | CD34 Separation | | | | |
|---|---|---|---|---|---|---|---|---|---|---|---|---|---|
| ID/No | BM [ml] | Age | Sex | Oriori [%] | Purity [%] | Total Cell number | Total RNA amount [ng] | RNA amount [ng] per 10^6 cells | Oriori [%] | Purity [%] | Total Cell number | Total RNA amount [ng] | RNA amount [ng] per 10^6 cells |
| 7 | 70 | 81 | F | 0,80 | 89,7 | 3.8E+06 | 6185 | 1619 | 1,15 | 81,4 | 6,2E+06 | 18752 | 3049 |
| 8 | 60 | 73 | M | 0,72 | 56,0 | 5.3E+06 | 5510 | 1040 | 1,38 | 70,2 | 3,8E+06 | 5152 | 1356 |
| 9 | 60 | 80 | F | 0,29 | 70,7 | 1.7E+06 | 999 | 602 | 0,81 | 72,1 | 1,1E+06 | 1796 | 1633 |
| 10 | 100 | 80 | F | 0,58 | 84,4 | 3.7E+06 | 5465 | 1493 | 0,28 | 82,3 | 6,0E+06 | 9925 | 1662 |
| 11 | 60 | 81 | F | 0,19 | 62,8 | 1.9E+06 | 1700 | 909 | 0,42 | 60,4 | 1,3E+06 | 2000 | 1538 |
| mean | - | 79 | - | 0,52 | 72,7 | 3,3E+06 | 3972 | 1133 | 0,81 | 73,2 | 3,7E+06 | 7525 | 1848 |

**Table 4. Spearman correlation coefficients for the miRNA copy number expression profiles. The miRNA copy numbers were calculated for all detected miRNAs (listed in Figure 3) and the coefficients were determined between the different samples.**

| | | CD133⁺ cells | | | | CD34⁺CD133⁻ cells | | | |
|---|---|---|---|---|---|---|---|---|---|
| | | Donor 7 | Donor 8 | Donor 10 | Donor 11 | Donor 7 | Donor 8 | Donor 10 | Donor 11 |
| CD133⁺ cells | Donor 7 | - | 0.93 | 0.97 | 0.94 | 0.94 | 0.92 | 0.91 | 0.84 |
| | Donor 8 | 0.93 | - | 0.92 | 0.88 | 0.90 | 0.90 | 0.85 | 0.81 |
| | Donor 10 | 0.97 | 0.92 | - | 0.96 | 0.92 | 0.93 | 0.94 | 0.89 |
| | Donor 11 | 0.94 | 0.88 | 0.96 | - | 0.90 | 0.90 | 0.93 | 0.93 |
| CD34⁺CD133⁻ cells | Donor 7 | 0.94 | 0.90 | 0.92 | 0.90 | - | 0.95 | 0.92 | 0.90 |
| | Donor 8 | 0.92 | 0.90 | 0.93 | 0.90 | 0.95 | - | 0.95 | 0.92 |
| | Donor 10 | 0.91 | 0.85 | 0.94 | 0.93 | 0.92 | 0.95 | - | 0.92 |
| | Donor 11 | 0.84 | 0.81 | 0.89 | 0.93 | 0.90 | 0.92 | 0.92 | - |

### Example 3: Cluster analysis of miRNA expressed in CD133⁺ and CD34⁺CD133⁻ cells

Next, a cluster analysis of the miRNAs expressed in CD133⁺ and CD34⁺CD133⁻ cells was performed. The cluster was obtained from array hybridizations of different bone marrow subpopulations (n=5) and leukapheresis samples (mPB, n=2) versus universal reference (UR). Those miRNAs were included where the net signal intensity of the sample miRNA was one fold over background in at least four of five donors in the CD133⁺ and/or CD34⁺CD133⁻ cell population and where the miRNA was present in the UR. The four subpopulations clustered separately as visualized in FIG. 3 and revealed distinct miRNA signatures for the different cell populations. As expected, the similarity between CD133⁺ and CD34⁺CD133⁻ cells was higher than between CD133⁺ and CD34⁻CD133⁻ cells which mainly consist of granulocytes (Neg -R) or RBCs (Neg +R).

The miRNA profiles of the CD133⁺ bone marrow samples and the CD133⁺ mobilized leukapheresis samples were highly similar with the notable exception of miR-451 and miR-144 expression that could only be detected in the samples derived from bone marrow and not in the leukapheresis samples.

### Example 4: Significance analysis of microarrays (SAM)

Significance analysis of microarrays (SAM) was performed to investigate the differences between CD133⁺ and CD34⁺CD133⁻ cells (FIG. 4A). Discriminatory gene analysis (DGA) was done by using the SAM (Significance Analysis of Microarrays) algorithm returning 18 miRNAs (false discovery rate (median < 0.0001%) differentially expressed between CD133⁺ and CD34⁺CD133⁻ cells. The resulting miRNAs were grouped by similarities in expression patterns using two-dimensional hierarchical average linkage clustering

The 18 miRNAs were significantly differentially expressed with three distinct expression signatures: 13 miRNAs (group 1 and 2) were significantly higher expressed in CD133⁺ compared to CD34⁺CD133⁻ cells. These 13 miRNAs could be divided into one group of miRNAs that were not expressed in CD34⁻CD133⁻ cells (group1) and another group where the expression in CD34⁻CD133⁻ cells was even higher than in CD133⁺ cells (group 2). Five miRNAs (group 3) were significantly lower expressed in CD133⁺ cells as in CD34⁺CD133⁻ cells.

These three expression signatures were observed with both populations of CD34⁻CD133⁻ cells. The miRNAs in group 1 seemed to be most specific for HSCs and HPCs as they were not expressed in the bulk of bone marrow cells. The ratios for the 18 miRNAs significantly differentially expressed between CD133⁺ and CD34⁺CD133⁻ cells are shown in Table 3.

**Table3. miRNAs significantly differentially expressed in CD133⁺ cells compared to CD34⁺CD133⁻. The ratios were calculated by dividing the ratio of sample signal and UR signal of the CD133⁺ arrays and CD34⁺CD133⁻ arrays and display the x-fold stronger or weaker expression of a miRNA in the CD133⁺ cells as compared to the CD34⁺CD133⁻ cells. miRBase accession = MIMAT accession number.**

| **miRNA name** | **miRBase accession** | **Donor 7** | **Donor 8** | **Donor 9** | **Donor 10** | **Donor 11** | **mean** |
|---|---|---|---|---|---|---|---|
| miR-10a | 0000253 | 3.2 | 5.1 | 8.8 | 3.2 | 8.0 | 6.3 |
| miR-125b | 0000423 | 3.2 | 4.8 | 6.4 | 2.4 | 5.3 | 4.7 |
| miR-146a | 0000449 | 2.6 | 3.2 | 3.5 | 2.1 | 6.7 | 3.9 |
| miR-125a-5p | 0000443 | 4.9 | 6.0 | 3.6 | 0.6 | 2.8 | 3.2 |
| miR-551b | 0003233 | 3.2 | 3.5 | 4.4 | 3.7 | 3.2 | 3.7 |
| miR-99a | 0000097 | 2.9 | 5.0 | 4.2 | 2.1 | 2.6 | 3.5 |
| miR-29a | 0000086 | 2.0 | 3.2 | - | 4.7 | 1.5 | 3.1 |
| miR-146b-5p | 0002809 | 2.1 | 2.2 | 1.9 | 2.1 | 2.2 | 2.1 |
| miR-29b | 0000100 | 1.5 | 3.4 | 2.0 | 2.4 | 0.7 | 2.1 |
| miR-29c | 0000681 | 1.5 | 3.0 | 2.2 | 1.7 | 1.0 | 2.0 |
| miR-23a | 0000078 | 1.3 | 1.9 | 1.9 | 1.8 | 0.9 | 1.6 |
| miR-24 | 0000080 | 1.4 | 1.5 | 1.6 | 1.8 | 1.1 | 1.5 |
| miR-23b | 0000418 | 1.7 | 1.5 | 1.2 | 1.8 | 1.0 | 1.4 |
| miR-142-5p | 0000433 | 0.9 | 0.7 | 0.9 | 0.5 | 0.5 | 0.7 |
| miR-191 | 0000440 | 0.8 | 0.8 | 0.7 | 0.5 | 0.6 | 0.6 |
| miR-142-3p | 0000434 | 1.0 | 0.4 | 0.8 | 0.5 | 0.6 | 0.6 |
| miR-484 | 0002174 | 0.5 | 0.5 | 0.4 | 1.2 | 0.4 | 0.6 |
| miR-425 | 0003393 | 0.6 | 0.5 | 0.5 | 0.5 | 0.3 | 0.4 |

### Example 5: absolute miRNA expression in CD133⁺ cells

The microarray data was also used to calculate the miRNA copy numbers per cell for the hematopoietic cell populations of four different donors. This absolute quantification was possible as the samples were hybridized versus the UR and analyzed according to a previously published method for absolute quantification (Bissels et al. 2009). The miRNA copy numbers were calculated for all detected miRNAs being also present in the UR and Spearman correlation coefficients were determined to assess the overall performance and to show the relationship between the different samples. The Spearman correlation coefficients of the miRNA copy number expression profiles were 0.88 - 0.97 for the CD133⁺ cells and 0.90 - 0.95 for the CD34⁺CD133⁻ cells. A comparison of the CD133⁺ cells with the CD34⁺CD133⁻ cells led to correlation coefficients between 0.81 - 0.94 (Table 4). Taken together, the correlation coefficients revealed a high similarity between different donors and a robust absolute measurement of miRNAs.

Next, the miRNA copy numbers were analyzed in detail. The majority of all detected miRNAs, 79% in CD133⁺ cells and 73% in CD34⁺CD133⁻ cells, were present with less than 200 copies per cell whereas the highest expressed miRNAs showed copy number of around 2,000 per cell (FIG. 4C). The most abundant miRNAs in CD133⁺ cells were miR-26a (7.2% of overall copy numbers), miR-451 (7.0%), and miR-26b (6.5%). The 25 highest expressed miRNAs in CD133⁺ and CD34⁺CD133⁻ cells accounted for 72.8% and 74.2%, respectively, of the overall miRNA pool (Table 5).

The significantly differentially expressed miRNAs (FIG. 4A) showed rather low expression levels with copy numbers below 200 copies per cell with exception of miR-142-3p with up to 5,000 copies per cell for one donor. FIG. 4B displays the copy numbers of three exemplary miRNAs representing the three different expression signatures of the differentially expressed miRNAs in CD133⁺ cells. miR-10a was expressed at highest level in CD133⁺ cells (group 1), miR-29a at high level in CD133⁺ and CD34⁻CD133⁻ cells (group 2) and miR-425 at lowest level in CD133⁺ cells (group 3).

**Table 5. 25 most abundant miRNAs detected in CD133⁺ and/or CD34⁺CD133⁻ cells. The percentages of the overall copy numbers for the most abundant miRNAs are shown, calculated as the median value of four independent measurements.**

| CD133 | | CD34 | |
|---|---|---|---|
| miRNAs | % | miRNAs | [%] |
| miR-26a | 7.2 | miR-142-3p | 8.5 |
| miR-451 | 7,0 | miR-451 | 5,9 |
| miR-26b | 6.5 | miR-223 | 5.4 |
| miR-142-3p | 5.7 | miR-26b | 5.1 |
| miR-16 | 4.7 | miR-16 | 4.8 |
| miR-101 | 4.6 | miR-101 | 4.6 |
| miR-19b | 4.2 | miR-26a | 4.5 |
| miR-223 | 4,0 | miR-19b | 4,5 |
| let-7f | 2.8 | miR-20a | 2.8 |
| miR-20a | 2.4 | miR-142-5p | 2.8 |
| miR-222 | 2.1 | let-7f | 2.6 |
| miR-92a | 2,0 | miR-92a | 2.1 |
| miR-19a | 2.0 | miR-19a | 2.1 |
| miR-142-5p | 2.0 | miR-15a | 2.0 |
| miR-15a | 1.9 | miR-30b | 2,0 |
| miR-30b | 1.9 | miR-21 | 2.0 |
| miR-21 | 1.9 | miR-144 | 1.9 |
| miR-126-3p | 1.5 | miR-126-3p | 1.8 |
| miR-92b | 1.4 | nuR-92b | 1.5 |
| let-7a | 1.3 | miR-222 | 1.4 |
| miR-144 | 1,3 | miR-17 | 1,4 |
| miR-17 | 1.2 | miR-181a | 1.1 |
| miR-29c | 1.1 | let-7a | 1.0 |
| miR-221 | 1.1 | miR-20b | 1.0 |
| miR-20b | 1.0 | let-7g | 1.0 |

### Example 6: Validation of miRNA data

The miRNA microarray results were validated using qRT-PCR that was performed for six arbitrary selected miRNAs (FIG. 5), four of which, miR-10a, miR-125a, miR-125b, miR-551b were expressed in higher levels and two of which, miR-425 and miR-484 were expressed in lower levels in CD133⁺ cells compared to CD34⁺CD133⁻ cells. The results correlated well with the microarray data.

### Example 7: In vitro characterization of functional miRNA and mRNA interactions

Luciferase reporter based assays were used to analyze some of the miRNA-mRNA interactions that were predicted by using bioinformatic tools. In detail, we cloned the respective miRNA binding site region of the FZD5, TPM1 and CD133 3'UTRs behind a luciferase reporter gene (FIG. 6 and FIG. 7). Direct interactions of miRNAs and putative target sites are then assessed based on selective degradation or translational repression of Luciferase-target gene 3'UTR fusion transcripts.

Cotransfection of 100 nM miR-29a and 300 ng pMIR-Luc-FZD5 decreased the luciferase activity by 40% and an even higher decrease of 60% was achieved by decreasing the vector amounts (FIG. 7). Besides miR-29a, binding sites for miR-24 and miR-99a were predicted within the FZD5 3'UTR. We also tested their potential function but a reproducible decrease in luciferase activity was not detectable for either of the tested miRNAs (data not shown).

The activity of pMIR-Luc-TPM1 was decreased by 35% upon cotransfection with miR-29a and again an even higher decrease of 58% was observed using lower vector amounts. The negative control using pMIR-Luc without cloned target sequence showed no decrease in luciferase activity after cotransfection with miR-29a.

The influence of miR-142-3p, that was described as hematopietic specific (Landgraf et al. 2007), on pMIR-Luc-CD133 was less pronounced, the luciferase activity was decreased by 20-30%. Interestingly, this effect was achieved reproducibly just whith 50 nM mir-142-3p mimic and ot with 100 nM miR-142-3p.

In conclusion, the luciferase assays indicated that FZD5, a receptor of the Wnt-signaling pathway, and tropomyosin (TPM1), which are lower expressed in CD133⁺ cells, are controlled by miR-29a. The expression of CD133 is probably regulated by the hematopoietic specific miRNA miR-142-3p.

### Example 8: Analysing transfection capability of miRNA into CD133⁺ cells

CD133⁺ cells were cultivated in StemSpan serum-free-medium supplemented with SCF, TPO and FGF-1. The cultivated cells were transfected with a FITC labeld miRNA mimic or with on oligonucleotide designed to serve as negative control (miR-Neg). The transfection efficiency was calculated in comparison to the negative control.

One day after transfection the cells were analyzed and over 50% of the cells were FITC positive, indicating a transfection efficiency of over 50 (Fig. 8A).

### Example 9: miRNA effect in CFU assay

The influence of some miRNAs - significantly differentially expressed in CD133⁺ cells compared to CD34⁺CD133⁻ cells - on the cultivation of CD133⁺ cells was tested. Therefore, the CD133⁺ cells were cultivated in StemSpan serum-free-medium supplemented with SCF, TPO and FGF-lin and transfected with miRNA mimics. Cells were seeded five days after transfection in HSC-CFU medium. After 14 days of cultivation the colony-forming ability was analyzed and revealed that the number of colonies was slightly lower for the cells transfected with miR-142-3p and miR-425 (Fig 8B). This was in accordance with the assumption that overexpression of miRNAs - lower expressed in CD133⁺ cells - pushed the cells towards CD133⁻ cells, leading to a lower colony-forming ability. On the contrary the colony-forming ability was higher for cells transfected with miR-125a-5p that is higher expressed in CD133⁺ cells. Further analysis revealed that CD133⁺ cells contacted with miR-142-3p showed a decrease in the overall colony forming ability of 71% (Fig. 8C) and that miR-425 decreased the percentage of CFU-M from 20% to 8.5 % (Fig. 8D).

### Example 10: miRNA effect in CFSE assay

The influence of miR-142-3p and miR-425 on proliferation of CD133+ cells was analyzed by performing the CFSE staining method. CD133⁺ cells were stained with CFSE and transfected with miR-142-3p, miR-425 or a control miRNA (miR-Neg) and cultivated for 6 days. The fluorescence dye CFSE is precisely halved at each cell generation and therefore proliferation is associated with reduced CFSE signal intensity. miR-142-3p as well as miR-425 led to a slightly but significantly decreased proliferation of CD133⁺ cells: The mean CFSE intensity of CD133⁺ cells was higher for cells transfected with miR-142-3p and miR-425 as compared to the negative controls (Fig. 9A). CD133+ cells transfected with miR-142-3p and miR-425 contained a lower proportion of cells that passed through more than two cell divisions compared to cells transfected with miR-Neg (Fig. 9B and C).

### Conclusion

Example 9 and 10 show that the differentiation status of cells, especially hematopoietic stem and progenitor cells e.g. CD133⁺ cells can be modulated by contacting the cells with RNA sequences corresponding substantially to the RNA sequences of the miRNAs as defined in the claims.

The cells obtain upon contact with the miRNAs as defined in the claims a lower or higher colony forming ability, depending on the miRNA used. A lower colony forming ability indicates that the differentiation status of the cells is changed from CD133+ to CD133- cells with reduced proliferation and reduced potential to generate different types of colonies, for example to form different types of blood cells. A higher colony forming ability indicates that the cells proliferate, maintain their primitive character as CD133+ cells (i.e. do not differentiate) and maintain their potential to generate different types of colonies.

### References

Bartel DP. 2009. MicroRNAs: target recognition and regulatory functions. Cell 136:215-233.
Bissels U, Wild S, Tomiuk S, Holste A, Hafner M, Tuschl T, Bosio A. 2009. Absolute quantification of microRNAs by using a universal reference. RNA 15:2375-2384.
Boxall SA, Cook GP, Pearce D, Bonnet D, El-Sherbiny YM, Blundell MP, Howe SJ, Leek JP, Markham AF, de Wynter EA. 2009. Haematopoietic repopulating activity in human cord blood CD133+ quiescent cells. Bone Marrow Transplant 43:627-635.
Buhring HJ, Seiffert M, Bock TA, Scheding S, Thiel A, Scheffold A, Kanz L, Brugger W. 1999. Expression of novel surface antigens on early hematopoietic cells. Ann N Y Acad Sci 872:25-38; discussion 38-29.
Copland M, Hamilton A, Elrick LJ, Baird JW, Allan EK, Jordanides N, Barow M, Mountford JC, Holyoake TL. 2006. Dasatinib (BMS-354825) targets an earlier progenitor population than imatinib in primary CML but does not eliminate the quiescent fraction. Blood 107:4532-4539.
de Wynter EA, Buck D, Hart C, Heywood R, Coutinho LH, Clayton A, Rafferty JA, Burt D, Guenechea G, Bueren JA et al. 1998. CD34+AC133+ cells isolated from cord blood are highly enriched in long-term culture-initiating cells, NOD/SCID-repopulating cells and dendritic cell progenitors. Stem Cells 16:387-396.
Eisen MB, Spellman PT, Brown PO, Botstein D. 1998. Cluster analysis and display of genome-wide expression patterns. Proc Natl Acad Sci U S A 95:14863-14868.
Feller N, van der Pol MA, Waaijman T, Weijers GW, Westra G, Ossenkoppele GJ, Schuurhuis GJ. 2005. Immunologic purging of autologous peripheral blood stem cell products based on CD34 and CD133 expression can be effectively and safely applied in half of the acute myeloid leukemia patients. Clin Cancer Res 11:4793-4801.
Friedman RC, Farh KK, Burge CB, Bartel DP. 2009. Most mammalian mRNAs are conserved targets of microRNAs. Genome Res 19:92-105.
Furst G, Schulte am Esch J, Poll LW, Hosch SB, Fritz LB, Klein M, Godehardt E, Krieg A, Wecker B, Stoldt V et al. 2007. Portal vein embolization and autologous CD133+ bone marrow stem cells for liver regeneration: initial experience. Radiology 243:171-179.
Gangaraju VK, Lin H. 2009. MicroRNAs: key regulators of stem cells. Nat Rev Mol Cell Biol 10:116-125.
Georgantas RW, 3rd, Hildreth R, Morisot S, Alder J, Liu CG, Heimfeld S, Calin GA, Croce CM, Civin CI. 2007. CD34+ hematopoietic stem-progenitor cell microRNA expression and function: a circuit diagram of differentiation control. Proc Natl Acad Sci USA 104:2750-2755.
Giebel B, Zhang T, Beckmann J, Spanholtz J, Wernet P, Ho AD, Punzel M. 2006. Primitive human hematopoietic cells give rise to differentially specified daughter cells upon their initial cell division. Blood 107:2146-2152.
Han YC, Park CY, Bhagat G, Zhang J, Wang Y, Fan JB, Liu M, Zou Y, Weissman IL, Gu H. 2010. microRNA-29a induces aberrant self-renewal capacity in hematopoietic progenitors, biased myeloid development, and acute myeloid leukemia. J Exp Med 207:475-489.
Hemmoranta H, Hautaniemi S, Niemi J, Nicorici D, Laine J, Yli-Harja O, Partanen J, Jaatinen T. 2006. Transcriptional profiling reflects shared and unique characters for CD34+ and CD133+ cells. Stem Cells Dev 15:839-851.
Isidori A, Motta MR, Tani M, Terragna C, Zinzani P, Curti A, Rizzi S, Taioli S, Giudice V, D'Addio A et al. 2007. Positive selection and transplantation of autologous highly purified CD133(+) stem cells in resistant/relapsed chronic lymphocytic leukemia patients results in rapid hematopoietic reconstitution without an adequate leukemic cell purging. Biol Blood Marrow Transplant 13:1224-1232.
Jin P, Wang E, Ren J, Childs R, Shin JW, Khuu H, Marincola FM, Stroncek DF. 2008. Differentiation of two types of mobilized peripheral blood stem cells by microRNA and cDNA expression analysis. J Transl Med 6:39.
Landgraf P, Rusu M, Sheridan R, Sewer A, Iovino N, Aravin A, Pfeffer S, Rice A, Kamphorst AO, Landthaler M et al. 2007. A mammalian microRNA expression atlas based on small RNA library sequencing. Cell 129:1401-1414.
Liao R, Sun J, Zhang L, Lou G, Chen M, Zhou D, Chen Z, Zhang S. 2008. MicroRNAs play a role in the development of human hematopoietic stem cells. J Cell Biochem 104:805-817.
Martinez HR, Gonzalez-Garza MT, Moreno-Cuevas JE, Caro E, Gutierrez-Jimenez E, Segura JJ. 2009. Stem-cell transplantation into the frontal motor cortex in amyotrophic lateral sclerosis patients. Cytotherapy 11:26-34.
Matsumoto K, Yasui K, Yamashita N, Horie Y, Yamada T, Tani Y, Shibata H, Nakano T. 2000. In vitro proliferation potential of AC133 positive cells in peripheral blood. Stem Cells 18:196-203.
Merkerova M, Vasikova A, Belickova M, Bruchova H. 2009. MicroRNA expression profiles in umbilical cord blood cell lineages. Stem Cells Dev 19:17-26.
Saeed AI, Sharov V, White J, Li J, Liang W, Bhagabati N, Braisted J, Klapa M, Currier T, Thiagarajan M et al. 2003. TM4: a free, open-source system for microarray data management and analysis. Biotechniques 34:374-378.
Stamm C, Kleine HD, Choi YH, Dunkelmann S, Lauffs JA, Lorenzen B, David A, Liebold A, Nienaber C, Zurakowski D et al. 2007. Intramyocardial delivery of CD133+ bone marrow cells and coronary artery bypass grafting for chronic ischemic heart disease: safety and efficacy studies. J Thorac Cardiovasc Surg 133:717-725.
Stamm C, Westphal B, Kleine HD, Petzsch M, Kittner C, Klinge H, Schumichen C, Nienaber CA, Freund M, Steinhoff G. 2003. Autologous bone-marrow stem-cell transplantation for myocardial regeneration. Lancet 361:45-46.
Summers YJ, Heyworth CM, de Wynter EA, Hart CA, Chang J, Testa NG. 2004. AC133+ G0 cells from cord blood show a high incidence of long-term culture-initiating cells and a capacity for more than 100 million-fold amplification of colony-forming cells in vitro. Stem Cells 22:704-715.
Sutherland DR, Anderson L, Keeney M, Nayar R, Chin-Yee I. 1996. The ISHAGE guidelines for CD34+ cell determination by flow cytometry. International Society of Hematotherapy and Graft Engineering. J Hematother 5:213-226.
Terstappen LW, Levin J. 1992. Bone marrow cell differential counts obtained by multidimensional flow cytometry. Blood Cells 18:311-330; discussion 331-312.
Tusher VG, Tibshirani R, Chu G. 2001. Significance analysis of microarrays applied to the ionizing radiation response. Proc Natl Acad Sci U S A 98:5116-5121.
Walenda T, Bork S, Horn P, Wein F, Saffrich R, Diehlmann A, Eckstein V, Ho AD, Wagner W. 2010. Co-culture with mesenchymal stromal cells increases proliferation and maintenance of haematopoietic progenitor cells. J Cell Mol Med 14:337-350.
Weigmann A, Corbeil D, Hellwig A, Huttner WB. 1997. Prominin, a novel microvilli-specific polytopic membrane protein of the apical surface of epithelial cells, is targeted to plasmalemmal protrusions of non-epithelial cells. Proc Natl Acad Sci U S A 94:12425-12430.
Xiao C, Rajewsky K. 2009. MicroRNA control in the immune system: basic principles. Cell 136:26-36.
Yin AH, Miraglia S, Zanjani ED, Almeida-Porada G, Ogawa M, Leary AG, Olweus J, Kearney J, Buck DW. 1997. AC133, a novel marker for human hematopoietic stem and progenitor cells. Blood 90:5002-5012.

## Claims

1. A method for modulating the differentiation of cells, comprising contacting said cells with one or more RNA sequences corresponding substantially to the RNA sequences of the miRNAs selected from the group consisting of miR-10a, miR-23a, miR-23b, miR-24, miR-29a, miR-29b, miR-29c, miR-99a, miR-125a-5p, miR-125b, miR-142-3p, miR-142-5p, miR-146a, miR-146b-5p, miR-191, miR-425, miR-484, miR-551b or the complement sequence thereof.

2. A method according to claim 1 wherein the cells are hematopoietic stem and progenitor cells.

3. A method according to claim 1 or 2 wherein the cells are CD133⁺ cells and/or progenitor cells and the modulation is the proliferation of said cells and wherein said cells maintain their self-renewal capability.

4. A method according to claim 1 or 2 wherein the cells are CD133⁺ cells and/or progenitor cells and the modulation is the direct differentiation of said cells.

5. A method according to claim 1 wherein the cells are more differentiated cells and the modulation is the dedifferentiation or reprogramming of said cells and wherein said cells are dedifferentiated or reprogrammed to primitive CD133⁺ cells and/or progenitor cells.

6. A cell culture medium comprising one or more RNA sequences corresponding substantially to the RNA sequences of the miRNAs selected from the group consisting of miR-10a, miR-23a, miR-23b, miR-24, miR-29a, miR-29b, miR-29c, miR-99a, miR-125a-5p, miR-125b, miR-142-3p, miR-142-5p, miR-146a, miR-146b-5p, miR-191, miR-425, miR-484, miR-551b or the complement sequence thereof.

7. The use of one or more RNA sequences corresponding substantially to the RNA sequences of the miRNAs selected from the group consisting of miR-10a, miR-23a, miR-23b, miR-24, miR-29a, miR-29b, miR-29c, miR-99a, miR-125a-5p, miR-125b, miR-142-3p, miR-142-5p, miR-146a, miR-146b-5p, miR-191, miR-425, miR-484, miR-551b or the complement sequence thereof for the modulation of cells.

8. The use according to claim 7, wherein the cells are CD133⁺ cells and/or progenitor cells and the modulation is the proliferation of said cells and wherein said cells maintain their self-renewal capability.

9. The use according to claim 7, wherein the cells are more differentiated cells and the modulation is the de-differentiation or reprogramming of said cells and wherein said cells are reprogrammed to primitive CD133⁺ cells and/or progenitor cells.

10. The use according to claim 7, wherein the cells are CD133⁺ cells and/or progenitor cells and the modulation is the direct differentiation of said cells.

11. A cell composition obtainable by the method according to any one of the claims 1 to 5

12. The use of the cell composition according to claim 11 for stem cell therapy.
